# FASCICULE DE BREVET EUROPEEN

(11) **EP 0 888 110 B1**
(45) Date de publication et mention de la délivrance du brevet: **17.10.2001**
(21) Numéro de dépôt: 97914410.2
(22) Date de dépôt: 14.03.1997
(51) Int. Cl.: A61K 9/16, A61K 9/51

(54) **MICROPARTICULES DE GEL COMPOSITE COMME VECTEURS DE PRINCIPES ACTIFS**
MIKROPARTIKEL AUS EINEM ZUSAMMENGESETZTEN GEL ALS WIRKSTOFFTRÄGER
COMPOSITE GEL MICROPARTICLES AS ACTIVE PRINCIPLE CARRIERS

(30) Priorité: 15.03.1996 FR 9603546
(43) Date de publication de la demande: 07.01.1999
(73) Titulaire: FLAMEL TECHNOLOGIES, 69693 Venissieux Cédex (FR)
(72) Inventeur: LEMERCIER, Alain, F-69720 Saint-Bonnet-de-Mûre (FR); MEYRUEIX, Rémi, F-69009 Lyon (FR); HUILLE, Sylvain, F-69008 Lyon (FR); SOULA, Gérard, F-69330 Meyzieu (FR)
(74) Mandataire: Fleurance, Raphael
(86) Numéro de dépôt international: FR9700471
(87) Numéro de publication internationale: WO9734584

(56) Documents cités:
- EP-A- 0 734 720
- GB-A- 2 260 080
- US-A- 4 976 968

## Description

Le domaine de la présente invention est celui des vecteurs utiles pour l'administration de principes actifs (**PA**), de préférence médicamenteux ou nutritionnels, notamment par voie orale, nasale, vaginale, oculaire, sous-cutanée, intraveineuse, intramusculaire, intradermique, intrapéritonéale, intracérébrale, parentérale, etc. Ces vecteurs permettent le transport sous protection des **PA,** à l'intérieur de l'organisme, jusqu'à leur site d'action. Ils visent à améliorer la biodisponibilité des **PA.** Ces vecteurs peuvent être, e. g., des systèmes à libération prolongée de **PA.**

Les **PA** plus particulièrement, mais non limitativement, concernés par l'invention sont, par exemple, des protéines, des glycoprotéines, des peptides, des polysaccharides, des lipopolysaccharides, des oligonucléotides et des polynucléotides.

La présente invention concerne, plus précisément, des particules - avantageusement de type submicroniques et/ou microniques - de Vectorisation (**PV**), de **PA,** en particulier médicamenteux.

Outre les **PA** médicamenteux et nutritionnels, l'invention concerne, également, des **PA** phytosanitaires ou cosmétiques.

La présente invention vise aussi bien les particules nues en tant que telles, que les systèmes de vecteurs de **PA,** constitués par les particules chargées par le (ou les) **PA** considéré(s).

L'invention concerne, également, un procédé de préparation desdites particules.

### ART ANTERIEUR :

Les progrès du génie génétique et des biotechnologies, ainsi que les découvertes y afférentes d'outils génétiques, de protéines et de peptides biologiquement actifs, ont permis l'essor de nouveaux principes actifs médicamenteux (**PA**) offrant une activité intrinsèque et une sélectivité élevées. Ces **PA** sont, en revanche, facilement dégradés dans l'organisme avant d'atteindre leur site d'action thérapeutique et leur biodisponibilité est, en conséquence, très faible. Dans le cas de l'administration par la voie orale, le tractus gastro-intestinal constitue une barrière chimique et physique redoutable pour le **PA** qui doit, d'une part, résister à la dégradation par le système digestif et, d'autre part, passer à travers la membrane épithéliale gastro-intestinale. A cet égard, on pourra, par exemple, se reporter à la revue de M. J. HUMPHREY (Delivery System for peptide Drugs, éditée par S. DAVIS et L. ILLUM, Plenum Press, N. Y., 1986), qui fait état de la faible biodisponibilité des peptides et des peptides administrés par voie orale.

Naturellement, ces avatars de transport et de séjour dans l'organisme ne se limitent pas aux protéines, mais affectent également les **PA** formés par des outils génétiques (oligonucléotides, polynucléotides, plasmides) susceptibles d'être mis en oeuvre dans les techniques de thérapie génique.

Pour pallier cela, il a été proposé d'encapsuler les **PA** dans des particules de vectorisation de **PA,** dénommées également **PV.** L'intérêt de ces techniques d'encapsulation est de protéger et/ou de transporter le **PA** jusqu'à son site d'action thérapeutique, en le sauvegardant contre les agressions de l'organisme, afin d'augmenter sa biodisponibilité.

Parmi tous les matériaux envisageables pour l'encapsulation de **PA,** les polymères sont de plus en plus utilisés, du fait de leurs propriétés intrinsèques.

S'agissant du cahier des charges que l'on souhaite obtenir pour de telles **PV,** il est particulièrement exigeant et comprend, notamment, les spécifications suivantes.
**1 -** Il devrait, avantageusement, être possible de pouvoir disposer de **PV** de diamètre moyen compris entre une fraction de micron et quelques microns, avec une répartition granulométrique étroite, de façon à pouvoir adapter la granulométrie des **PV** au mode d'administration choisi et/ou le site thérapeutique visé. Par exemple, si une immunisation mucosale par la voie orale est recherchée, la taille des **PV** doit être comprise entre 0,5 µm et 10 µm, afin que les **PV** puissent pénétrer les plaques de Peyer et atteindre les tissus lymphoïdes. Dans le cas d'une administration sous-cutanée, il y a avantage à disposer de **PV** de taille supérieure à 10 µm pour que les particules ne rentrent pas dans la circulation générale où elles sont rapidement internalisées par le système réticulo-endothélial, mais qu'elles diffusent progressivement depuis leur site d'injection.
   Cette spécification implique un contrôle dimensionnel des **PV,** à la fois sur la distribution de la granulométrie des **PV** et sur leur diamètre moyen, qui représente une opération très délicate sur le plan technologique.
**2 -** Il est souhaitable que les **PV** assurent la protection du **PA** jusqu'au site de libération. Par exemple, dans une administration orale d'un **PA** formé par un vaccin, ce dernier gagnerait à être protégé, tout au long du tractus gastrointestinal.
**3 -** Il est préférable que le polymère, constituant les **PV,** soit biocompatible et biodégradable et, encore mieux, qu'il soit métabolisé en produits non toxiques pour l'organisme.
**4 -** Il est également avantageux que le polymère, constitutif des **PV,** n'induise pas de réponse immunitaire (immunogéniale).
**5 -** On attend des **PV** qu'elles permettent la libération contrôlée et prolongée du **PA.**
**6 -** Enfin, il est également préférable que les **PV** puissent être obtenues par un procédé non dénaturant pour le **PA.** Ainsi, l'usage de solvants organiques et/ou de températures élevées est à proscrire.

De nombreuses propositions techniques antérieures ont vainement tenté de satisfaire à l'ensemble de ces spécifications. Les réponses apportées jusqu'alors ne sont donc que partielles et incomplètes.
Dans toutes ces propositions infructueuses de l'art antérieur, plusieurs matériaux constitutifs de base ont été envisagés pour les particules de vectorisation **PV.** Ces matériaux peuvent être des polymères biocompatibles, tels que les protéines et/ou les polymères d'α-hydroxyacides (polylactiques et/ou glycoliques) et/ou des polycyanoacrylates d'alkyle et/ou des polyorthoesters et/ou des corps gras (huiles - graisses).

Les **PV** (microsphères ou microcapsules) de nature protéique sont, la plupart du temps, obtenues par des traitements drastiques de réticulation à l'aide d'agents chimiques du type glutaraldéhyde ou par élévation de la température. Il va sans dire que de tels traitements entraînent nécessairement une dénaturation de bon nombre de principes actifs. De surcroît, la toxicité des agents réticulants du type glutaraldéhyde est particulièrement mal venue dans les applications pharmaceutiques. A titre d'exemples de telles microparticules connues, on peut citer celles divulguées dans la demande de brevet EP 0 363 921. Les particules selon cette demande sont obtenues par coacervation complexe d'un polyaminoacide synthétique et d'un polymère anionique, en solution aqueuse, par ajustement du pH. Le polyaminoacide est un copolymère amphiphile à base d'acide glutamique et de lysine, tandis que le polymère anionique est un polysaccharide soluble dans l'eau, tel que la gomme arabique. On donne de la cohésion à ces particules par réticulation du coacervat à l'aide de glutaraldéhyde. Outre les problèmes de toxicité et de dégradation des **PA** signalés ci-dessus, il convient d'observer que les polymères du coacervat selon l'EP 0 363 921 souffrent d'un caractère immunogène.

Des microparticules, protéiques ou à base de polymères biocompatibles et biodégradables, peuvent également être préparées par les techniques classiques d'émulsion.
A ce propos, on peut évoquer, par exemple, les demandes de brevets WO 91/06 286 et WO 91/06 287 qui décrivent des procédés de formation de particules en émulsion dans lesquels on utilise, comme polymère :
- soit une protéine hydrophobe choisie parmi le collagène, la caséine, la kératine et, de préférence, les prolamines,
- soit un polymère biocompatible et biodégradable, tel que les poly(lactiques) ou les poly(orthoesters).
Le **PA** peut être hydrophobe ou hydrophile mais, dans ce dernier cas, la technique de double émulsion est recommandée. La taille des microparticules est d'environ 100 µm et, de préférence, comprise entre 50 nm et 100 µm.

La demande de brevet WO 89/08 449 fait également référence à l'encapsulation par émulsion, pour incorporer des **PA** dans des microparticules de poly(lactiques) de taille inférieure à 10 µm. Et il est précisé, dans ce document, que cette taille est une limite maximale pour l'absorption au travers des tissus lymphoïdes des muqueuses (administrations orale, nasale, rectale, ophtalmologique).
Les techniques d'émulsion sont très séduisantes a priori, car elles permettent la mise en oeuvre de la plupart des **PA** dans des microparticules, dont on peut contrôler la granulométrie jusqu'à des tailles de l'ordre de 1 µm. Mais, dans ces techniques, on a recours à des solvants organiques pour solubiliser les polymères constitutifs des particules. Ces solvants sont, e. g. des cétones, des alcools, des amides ou leurs mélanges. Et malheureusement, il s'avère que ces solvants peuvent être dénaturants, notamment pour les **PA** peptidiques ou polypeptidiques.
On signalera, également, à la charge des polymères constitutifs du type poly-α-hydroxyacides (polylactique et/ou glycolique) un problème d'accumulation in vivo, de nature à engendrer des effets de rejet.

On connaît, également, des **PV** biocompatibles, formées en solution aqueuse sans élévation excessive de la température et appelées protéinoïdes. Ces **PV** ont été décrites dès 1970 par W. FOX et K. DOSE dans "Molecular Evolution and the origin of Life", Ed. Marcel DEKKER Inc. (1977).
En s'inspirant de ces travaux, la demande de brevet WO 88/01 213 propose un système à délivrance de **PA** à base de protéinoïdes. Le polymère utilisé est un mélange de polypeptides artificiels obtenus par condensation thermique d'acides aminés synthétiques ou naturels et/ou de petites chaînes peptidiques. Le mode de condensation choisi conduit à des oligomères ramifiés et donc très peu solubles. Il est ensuite procédé à une sélection par filtration de ces oligomères ramifiés, afin de récupérer les fractions hydrosolubles. Cette fraction est nécessairement composée de réticulats ramifiés de très petite masse. Les microparticules selon cette invention sont obtenues par changement de pH qui crée la précipitation des oligomères ramifiés en protéinoïdes.
Lorsque la solution dans laquelle s'effectue la précipitation contient des **PA** en solution, une partie d'entre eux est entraînée dans le protéinoïde lors de sa formation.
Les inconvénients de ce système sont :
- un faible taux d'encapsulation,
- une méthode de synthèse par abaissement de pH,
- un procédé de purification des polymères délicat,
- un enchaînement non régulier (non alpha peptidique) des aminoacides dû au mode de synthèse qui ne permet pas d'affirmer que les réactions de dégradation enzymatiques seront identiques à celles d'un alpha-polyaminoacide,
- enfin, l'utilisation d'un grand nombre d'aminoacides monomères différents, qui peut induire une réponse immunitaire.

La demande de brevet WO 93/25 589 porte sur l'amélioration du procédé de synthèse de proténoïdes par condensation thermique d'acides aminés. On retrouve les mêmes inconvénients que ceux attachés à l'objet de la demande mère évoquée ci-dessus.

Les microparticules, du type nanocapsules décrites dans la demande de brevet EP 0 608 207 et dont la paroi est à base de poly(2-cyanoacrylate d'alkyle), ne sont citées ici que pour mémoire, dans la mesure où la toxicité des résidus de monomères mis en oeuvre est clairement rédhibitoire pour les vectorisations de **PA** chez l'homme et/ou l'animal. Par ailleurs, on connaît mal l'élimination des résidus de tels polymères.

S'agissant maintenant des **PV** de nature essentiellement lipidique ou hydrophobe, on peut dénombrer plusieurs types de systèmes, à savoir
- les liposomes,
- les particules lipidiques solides (SLP),
- les vésicules lipidiques à multiples chambres (VLMC),
- les particules de fondu super-refroidi (PSM),
- les émulsions lipidiques,
- et les particules du type porteur matriciel lipidique (LMC).

Les liposomes sont des structures colloïdales sphériques comprenant une phase interne aqueuse enveloppée par un ou plusieurs bi-couches de phospholipides. Les liposomes sont connus en tant que particules de vectorisation.
Un premier inconvénient des liposomes est leur instabilité dans les fluides biologiques et la vitesse élevée de relargage du **PA** qu'ils sont susceptibles de contenir. On se référera, à ce propos, à l'article de KIM et al., Biochim. Biophys. Acta, 728, 339-348, 1983.
Un deuxième inconvénient des liposomes est qu'ils ne permettent qu'un faible taux de chargement en **PA.**
Un troisième inconvénient des liposomes tient à leur instabilité au stockage.
Un quatrième inconvénient des liposomes est lié à la faible reproductibilité de leur fabrication et, notamment, à leur médiocre aptitude au piégeage de **PA.**
La littérature brevets sur les liposomes est dense. On citera, pour mémoire, les brevets suivants : US 3 993 754, US 4 235 871, US 4 356 167 et US 4 377 567.

La demande de brevet internationale PCT WO 94/20 072 décrit des particules lipidiques solides (SLP), de forme non sphérique et constituées par une matrice lipidique cristalline, solide à température ambiante. Les lipides à haut point de fusion concernés sont, de préférence, des triglycérides (θ fusion = 30-120 °C). En suspension, ces SLP peuvent être stabilisées par des composés amphiphiles, ioniques ou non ioniques. Ces composés amphiphiles stabilisants peuvent être des phospholipides, des sphingolipides, des glycosphingolipides, des sels biliaires physiologiques, des acides gras saturés ou non, des alcools gras, des acides ou des alcools gras méthoxylés, de même que leurs esters et leurs éthers, des alcools alkyloaryles polyéthers, des esters et des éthers de sucres ou de sucres-alcools avec des acides gras ou des alcools gras, des mono ou des di-glycérides acétylés ou éthoxylés, des copolymères blocs de polyoxyéthylène et de polyoxypropylène oxyde, des éthers ou des esters de sorbitan éthoxylé, des aminoacides, des polypeptides, des protéines (gélatines, albumines), ainsi que les mélanges des susdits composés.
Du fait de leur nature solide cristalline, ces SLP comportent, nécessairement, une étape de fusion à température élevée, lors de leur formage et de l'incorporation de **PA.** Il a déjà été signalé que les chauffages à températures élevées sont nuisibles à certains **PA** sensibles. En outre, compte tenu de la nature exclusivement lipidique des SLP, ces derniers s'avèrent non adaptés pour les **PA** hydrophiles. Enfin, lorsqu'il s'agit d'un **PA** hydrophobe, de nature différente de celle formant la matrice du SLP, les rendements d'incorporation sont extrêmement faibles, de l'ordre de quelques %.

La demande de brevet internationale PCT WO 95/13 796 décrit des vésicules lipidiques à multiples chambres aqueuses internes (VLMC). Ces **PV** trouvent leur originalité par rapport aux liposomes, au travers de leur structure à multichambres non concentriques et à contenu aqueux. Lesdites chambres aqueuses sont formées chacune par une membrane formée d'un bicouche lipidique définissant une sphère.
Pour préparer ces VLMC, on met en oeuvre un lipide neutre, du type huile végétale, graisse animale ou tocophérol, ainsi qu'un lipide amphiphile doté d'une forte charge négative - e. g. phosphatidylsérine -. Ces deux types de lipides sont mis en solution dans un solvant organique auquel on ajoute une solution aqueuse comprenant le **PA** à encapsuler, de manière à former ainsi une émulsion eau dans huile. On complète cette émulsion par ajout d'un agent retardant la libération du **PA.** On procède ensuite à une deuxième émulsion par addition d'une solution aqueuse secondaire contenant au moins un agent osmotique non-ionique et un agent neutralisant les acides et ayant une faible force ionique. Après agitation, il se forme des sphérules de solvant contenant de multiples gouttelettes aqueuses. Par évaporation du solvant, on transforme ces sphérules en VLMC. Les chambres aqueuses internes sont donc en suspension dans la solution aqueuse secondaire et non dans le chloroforme qui a été éliminé.
Comme ses alter ego, cette technique de double émulsion est critiquable en ce qu'elle fait intervenir des solvants organiques toxiques, dont on ne peut garantir l'absence de traces dans les microparticules finales.

La demande de brevet internationale PCT WO 95/05 164 décrit des particules d'ubidecarenone ou d'autres substances faiblement solubles dans l'eau, dans lesquelles cette ou ces substances faiblement solubles dans l'eau, sont à l'état de "fondu super-refroidi", c'est-à-dire qu'elles se présentent dans un état assimilable à l'état liquide à une température inférieure à leur température de fusion. Ces particules, dénommées également PSM, requièrent la mise en oeuvre d'un stabilisant amphiphile (lécithine) lorsqu'elles sont mises en suspension dans un liquide aqueux Ces stabilisants sont les mêmes que ceux décrits dans la demande PCT WO 94/20 072 portant sur les SLP. Le fondu super-refroidi constituant la particule possède une température de fusion de l'ordre de 70 °C. Il s'agit donc de graisses et non pas d'huiles liquides à température ambiante. Les corps gras, susceptibles de former un tel fondu, sont choisis parmi les vitamines, les stérols ou les triglycérides, par exemple.
Ces PSM sont obtenus par fusion de la substance destinée à former le fondu super-refroidi. Le composé amphiphile est alors dispersé dans cette substance fondue. On ajoute ensuite de l'eau et l'on soumet le mélange à une homogénéisation/agitation à haute vitesse, tout en maintenant la température réactionnelle au dessus de la température de fusion de la substance mise en oeuvre. On obtient in fine une dispersion de PSM dans une phase aqueuse continue. L'eau est absente, ou quasiment absente, des microparticules de PSM.
De tels vecteurs ont l'inconvénient d'être spécifiques et exclusifs aux **PA** hydrophobes. En outre, les températures élevées, requises pour la conduite du procédé de préparation des ces PSM, peuvent être préjudiciables aux **PA.**

On connaît, également, des systèmes de vectorisation faisant intervenir des émulsions lipidiques constituées par des gouttelettes d'huile liquide dispersées dans une phase aqueuse et stabilisées par un film interfacial d'émulsifiant (lécithines). La vectorisation de **PA** à l'aide de **PV,** constituant la phase hétérogène d'émulsion lipidique, est décrite dans la demande de brevet international PCT WO 91/02 517. La cohésion, l'intégrité ou la stabilité de tels **PV** est précaire ou fragile. En effet, ces **PV** n'ont pas d'existence physique, i. e. ils ne forment pas de corps certain, en dehors du milieu d'émulsion. Dès lors que l'équilibre instauré par le tensioactif est perturbé, les gouttelettes d'huile liquide coalescent et disparaissent. En conséquence, ces émulsions lipidiques n'offrent que très peu de débouchés dans la vectorisation de **PA.**

Le brevet US 4 610 868 concerne un nouveau type de **PV** lipidique, qui peut être décrit comme étant une structure globulaire formée par une matrice lipidique. Ces **PV,** également dénommés LMC, ont une taille comprise entre 0,5 µm et 100 µm. Les constituants de base des LMC sont un composé hydrophobe, un composé amphiphile et, éventuellement, un **PA** accompagné d'eau si ce dernier est de nature hydrosoluble.
Le composé hydrophobe est, de préférence, une huile à base de triglycéride, telle que l'huile de maïs ou l'huile de noix de coco. Les stérols peuvent également convenir comme composés hydrophobes.
Les composés amphiphiles préconisés sont ceux de nature lipidique, tels que les phosphoglycérides et, plus particulièrement, la phosphatidylcholine du jaune d'oeuf.
La préparation de ces LMC fait intervenir un solvant organique, tel que l'acétone ou l'éthanol. Selon un mode particulier de mise en oeuvre du procédé de préparation de ces LMC, une émulsion eau dans huile - obtenue à partir d'huile de phosphatidylcholine, d'eau et de **PA** hydrosolubles - est extrudée dans le solvant organique sous agitation. Les LMC sont générés au moment de l'introduction de l'émulsion E/H dans le solvant. Dans le cas où le **PA** est hydrophobe, le mélange d'huile amphiphile et de **PA** s'opère dans le solvant organique, ledit mélange étant ensuite extrudé dans une phase aqueuse.
Pour les **PA** solubles dans l'eau, il est envisageable d'éliminer totalement la phase aqueuse et d'extruder le mélange huile + amphiphile + **PA** dans le solvant organique. Après élimination du solvant organique ou aqueux, on récupère des LMC qui, selon le brevet, sont décrites comme ressemblant à des gouttelettes d'huile qui ne coalescent pas.
Ces LMC présentent l'inconvénient majeur de nécessiter la mise en oeuvre de solvants lors de leur préparation et l'on sait que la présence de solvant, même à l'état de traces dans les **PV,** est particulièrement indésirable. On soulignera la nature exclusivement lipidique des LMC. Enfin, il doit être considéré que le procédé d'obtention des LMC est relativement complexe et peu commode à mettre en oeuvre de façon industrielle (extrusion dans un solvant).

Dans cet état de connaissances, l'un des objectifs essentiels de la présente invention est de fournir des **PV,** en particulier submicroniques et microniques, à base de lipides et susceptibles de servir de vecteurs d'un principe actif (**PA**), en particulier médicamenteux et/ou nutritionnel, pour l'administration dudit **PA** à un organisme humain ou animal, ces **PV** satisfaisant pleinement au cahier des charges explicité supra et répété ci-après :
**1 -** Il devrait, avantageusement, être possible de pouvoir disposer de **PV** de diamètre moyen compris entre une fraction de micron et quelques microns, avec une répartition granulométrique étroite, de façon à pouvoir adapter la granulométrie des PV au mode d'administration choisi et/ou le site thérapeutique visé. Par exemple, si une immunisation mucosale par la voie orale est recherchée, la taille des **PV** doit être comprise entre 0,5 µm et 10 µm, afin que les **PV** puissent pénétrer les plaques de Peyer et atteindre les tissus lymphoïdes. Dans le cas d'une administration sous-cutanée, il y a avantage à disposer de **PV** de taille supérieure à 10 µm pour que les particules ne rentrent pas dans la circulation générale où elles sont rapidement internalisées par le système réticulo-endothélial, mais qu'elles diffusent progressivement depuis leur site d'injection.
   Cette spécification implique un contrôle dimensionnel des **PV,** à la fois sur la distribution de la granulométrie des **PV** et sur leur diamètre moyen, qui représente une opération très délicate sur le plan technologique.
**2 -** Il est souhaitable que les **PV** assurent la protection du **PA** jusqu'au site de libération. Par exemple, dans une administration orale d'un **PA** formé par un vaccin, ce dernier gagnerait à être protégé, tout au long du tractus gastrointestinal.
**3** - Il est préférable que le polymère, constituant les **PV,** soit biocompatible et biodégradable et, encore mieux, qu'il soit métabolisé en produits non toxiques pour l'organisme.
**4 -** Il est également avantageux que le polymère, constitutif des **PV,** n'induise pas de réponse immunitaire (immunogéniale).
**5 -** On attend des **PV** qu'elles permettent la libération contrôlée et prolongée de **PA.**
**6 -** Enfin, il est également préférable que les **PV** puissent être obtenues par un procédé non dénaturant pour le **PA.** Ainsi, l'usage de solvants organiques et/ou de températures élevées ou des modifications drastiques de pH sont à proscrire.

Un autre objectif essentiel de l'invention est de fournir des PV à base de lipides qui soient stables au stockage, quel que soit le milieu extérieur, c'est-à-dire qui gardent leur intégrité physique, aussi bien dans une phase continue formée par de l'eau, par une solution aqueuse ou par un solvant organique, de sorte qu'elles ne doivent pas avoir tendance à coalescer entre elles.

Un autre objectif essentiel de l'invention est de fournir des **PV** à base de lipides qui soient d'une granulométrie moyenne contrôlable et ajustable.

Un autre objectif essentiel de l'invention est de fournir des **PV** qui soient simples à préparer (pH non agressif), stables à tout pH compris entre 2 et 13 et non immunogènes.

Un autre objectif essentiel de l'invention est de fournir des **PV** à base de lipides qui soient faisables industriellement et économiques et qui soient aptes à se charger en **PA** hydrophile tout comme lipophile avec des forts taux de chargement.

Un autre objectif essentiel de l'invention est de fournir un procédé de préparation de **PV** à base de lipides et susceptibles d'être utilisées comme vecteurs de **PA,** ledit procédé se devant d'être économique, simple à mettre en oeuvre, non dénaturant pour les **PA** et devant, en outre, permettre une maîtrise fine de la granulométrie moyenne des particules obtenues.

Un autre objectif essentiel de l'invention est l'utilisation des susdites particules pour la préparation de médicaments (e. g. vaccins) et/ou de nutriments, en particulier pour administration *per os,* nasale, vaginale, oculaire, sous-cutanée, intraveineuse, intramusculaire, intradermique, intrapéritonéale, intracérébrale ou parentérale, de principes actifs, tels que des protéines, des glycoprotéines, des peptides, des polysaccharides, des lipopolysaccharides, des oligonucléotides et des polynucléotides.

Un autre objectif essentiel de l'invention est de fournir un médicament du type système à libération prolongée de **PA,** qui soit biocompatible et qui procure une haute biodisponibilité du **PA.**

### BREF EXPOSE DE L'INVENTION :

Les objectifs relatifs aux produits, parmi d'autres, sont atteints par la présente invention qui concerne des particules, notamment de vectorisation de principe(s) actif(s), du type de celles à base de microparticules susceptibles d'être utilisées comme vecteurs de principe(s) actif(s) (**PA**), caractérisées
* α * en ce qu'elles ont chacune une structure cohésive faite de gel-composite, physico-chimiquement stable et intègre,
* β * en ce qu'elles tirent leur cohésion de la présence des trois composés suivants :
   **(I)** de l'huile,
   **(II)** une phase aqueuse,
   **(III)** et au moins un copolyaminoacide linéaire non réticulé, synthétique et comportant au moins deux types différents de comonomères aminoacides AAᵢ hydrophile et AAₒ hydrophobe,
* γ * et en ce qu'elles présentent une taille moyenne contrôlable et ajustable sur une gamme inférieure ou égale à 500 µm, de préférence à 200 µm et, plus préférentiellement, comprise entre 0,05 et 100 µm.

Il est du mérite de la demanderesse d'avoir mis au point ces microparticules en partie lipidiques, en procédant, d'une part, à une sélection de produits constitutifs particuliers, à savoir huile, eau et copolyaminoacide spécifique, et, d'autre part, en mettant au point un protocole d'obtention desdites microparticules qui permet, de manière tout à fait surprenante et inattendue, de conduire à une suspension colloïdale de particules cohésives bien différenciées et extrêmement stables.

La stabilité est bien l'une des caractéristiques originales les plus étonnantes des particules selon l'invention. Celles-ci conservent, en effet, leur intégrité et leur caractère différencié, aussi bien dans un milieu liquide, quel que soit le pH du milieu, qu'à l'état de lyophilysat. En d'autres termes, cela signifie qu'elles peuvent être isolées par filtration ou décantation (par centrifugation e. g.), sans coalescer. Elles forment donc des entités de type gel composite, qui possèdent leur identité et leur existence propre, quel que soit leur milieu de stockage.

Au sens de la présente invention, le terme "gel composite" désigne ou fait référence à une notion de gel physique à base d'eau, d'huile et de polymère.

Les **PV** selon l'invention sont, par ailleurs, des biovecteurs, aptes à inclure tout principe actif, quelle que soit sa nature hydrophile ou hydrophobe, et à assurer son transport à l'intérieur de l'organisme. Ces **PV** sont des biovecteurs d'autant plus convenables :
- qu'ils sont non immunogènes, biodégradables et exempts de produits toxiques de structuration et/ou de fabrication, et ce même à l'état de traces (solvants, glutaraldéhyde),
- et que leur taille (granulométrie) est contrôlable sur une large gamme.

En définitive, les **PV** sont des microparticules stables et cohésives. Elles ne coalescent pas, à la différence des gouttelettes d'émulsion.

### DESCRIPTION DETAILLEE DE L'INVENTION :

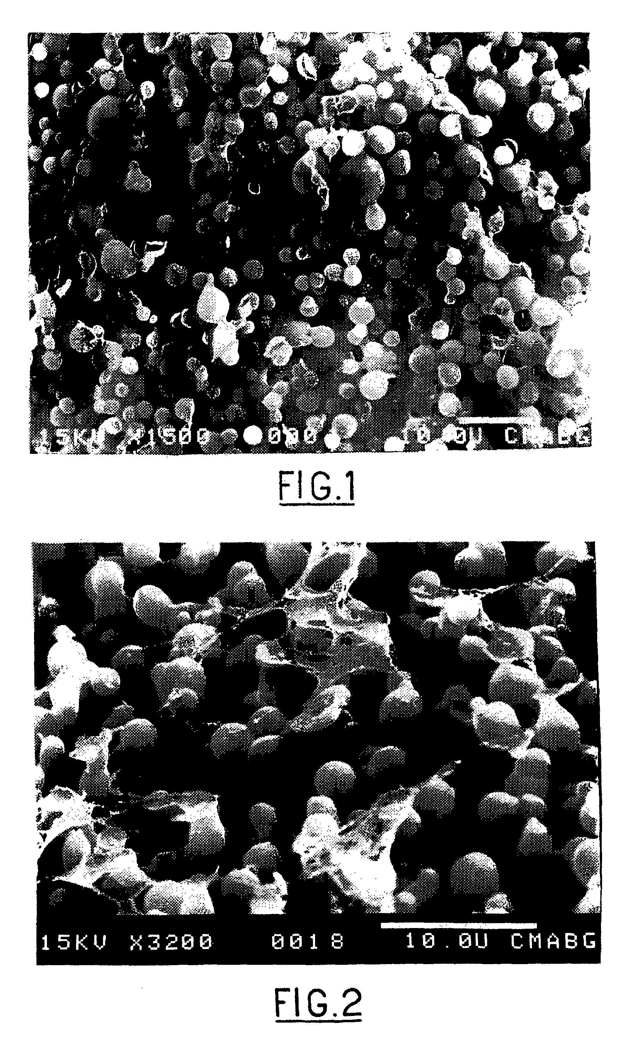

La morphologie de ces **PV** cohésives selon l'invention, apparaît clairement sur les photographies en Microscopie Electronique à Balayage (MEB) données aux **fig. 1** et **2** annexées. La description de ces photographies MEB est reprise ci-après, notamment dans les exemples. Mais on peut, d'ores et déjà, remarquer que ces **PV** de gel-composite sont de forme sensiblement sphérique, d'aspect lisse. Elles sont bien différenciées les unes des autres.

S'agissant de leur structure, il est possible d'indiquer qu'il ne s'agit pas d'une organisation en feuillets du type de celle des liposomes, pas plus que de simples gouttelettes de phase hétérogène d'émulsion H/E ou E/H.

A défaut d'éléments tangibles de caractérisation de leur structure, la demanderesse a pu définir ces **PV** de gel-composite au travers de particularités fonctionnelles et, plus précisément, de comportement de ces **PV** des tests d'évaluation technique discriminants.

Ainsi, les caractéristiques * α * des **PV** visées ci-dessus sont reflétées par au moins l'une des propriétés fonctionnelles suivantes :
- α₁ - absence de coalescence des microparticules suite à un traitement de lyophilisation, cette non-coalescence se traduisant par une conservation de la répartition granulométrique, en particulier du D[4,3] à raison de ± 20 %, après réhydratation selon un test α₁ ;
- α₂ - absence de coalescence à la centrifugation se traduisant par une conservation de la répartition granulométrique et, en particulier du D[4,3] à raison de ± 20 %, selon un test α₂ ;
- α₃ - résistance aux variations de pH se traduisant par une conservation de la répartition granulométrique des microparticules et, en particulier, du D[4,3] à raison de ± 20 %, après soumission des pH de 3 et de 13, selon un test α₃ ;
- α₄ - absence de coalescence en dispersion dans une solution aqueuse tamponnée, se traduisant par une conservation de la répartition granulométrique des microparticules, en particulier de leur D[4,3] à raison de ± 20 %, pendant des durées de stockage supérieures ou égales à 3, 9 et 18 mois à des températures de 37 °C, ambiante et 4 °C respectivement, selon un test α₄.

Les tests, α₁, α₂, α₃, α₄, dont il est question supra, sont utiles comme normes de définition dans le cadre du présent exposé. Ces tests sont définis en détail ci-après.
- *Test α*_{*1*} *de stabilité des* ***PV*** *à la lyophilisation/réhydratation :*
   Dans un ballon de 500 ml, on prépare 50 ml d'une suspension de **PV** à 0,2 % en poids, calculé sur la base de la masse de polymère constituant les **PV.** La phase aqueuse saline tamponnée employée est constituée par du PBS 0,01 M (tampon phosphate, pH 7,4 à 25 °C).
   Cette solution est ensuite congelée dans de l'azote liquide avant d'être lyophilisée à l'aide d'un dispositif de lyophilisation de marque CHRIST-ALPHA1-4 en soumettant l'échantillon à une pression de 20 Pa, à une température de - 52 °C pendant 48 h.
   On hydrate ensuite le lyophilisat ainsi obtenu en le mettant en présence d'un volume de phase aqueuse identique à celle utilisée pour la mise en suspension et représentant 5 ml.
   La répartition granulométrique des **PV** ainsi réhydratées est mesurée par diffraction laser à l'aide d'un COULTER LS 130 selon un modèle de calcul FRAUNHOFER avec "PIDS". Cela permet d'obtenir le D[4,3].
   On effectue une même mesure de répartition granulométrique avec détermination du D[4,3] avec la suspension de **PV** préparée ci-dessus et on compare les D [4,3] obtenus avec et sans lyophilisation/réhydratation.
- *Test α*_{*2*} *de stabilité des* ***PV*** *à la centrifugation :*
   On réalise une suspension de **PV** de la même façon que celle selon le text α₁ décrit ci-dessus, à la différence près que la concentration des **PV** dans la suspension est de 2 % en poids au lieu de 0,2 %.
   On centrifuge un échantillon de 1,5 ml de la susdite suspension à l'aide d'une centrifugeuse de marque SIGMA 3K30, pendant 10 min à 60 000 g.
   L'échantillon est agité manuellement pour remettre en suspension les **PV,** après centrifugation.
   On réalise les mesures granulométriques (D[4,3]) comme décrit dans le test α₁ ci-dessus. On compare les D[4,3] obtenus avant et après centrifugation.
- *Test α*_{*3*} *de résistance au pH des* ***PV*** *:*
   On introduit 0,5 ml de suspension de **PV** à 2 % (concentration exprimée de la même facon que celle donnée au test α₁) dans 5 ml d'une solution d'HCl titrant 10⁻² N (pH 2).
   On stocke une journée à température ambiante.
   La suspension est neutralisée par introduction de 50 µl de soude 1 N. On complète à 10 ml avec une solution saline tampon phosphate (PBS), pH 7,4.
   On réalise les mesures granulométriques de D[4,3] sur des suspensions ayant et celles n'ayant pas subi d'abaissement du pH à une valeur de 2. On compare les D[4,3] obtenus dans les deux cas de figures.
   On réalise exactement la même opération, mais cette fois en introduisant 0,5 ml de suspension de **PV** à 2 % (concentration exprimée de la même façon que celle donnée au test α₁) dans 5 ml de soude à 10⁻¹ N. On neutralise par addition de 0,5 ml d'HCl 1 N. On complète à 10 ml avec une solution saline tampon phosphate isotonique (PBS), pH 7,4.
   On procède aux mêmes mesures et aux mêmes comparaisons granulométriques de D[4,3].
- *Test α*_{*4*} *de stabilité au stockage des* ***PV*** *:*
   On réalise une suspension de **PV** en tous points identique à celle préparée conformément au test α₂ décrit ci-dessus.
   Des échantillons de cette suspension (2 ml) sont stockés à des températures de 2, 4 et 37 °C et à température ambiante.
   On effectue un suivi de la répartition granulométrique et, en particulier, du D[4,3] (même méthode de mesure que pour les tests α₁ à α₃ ci-dessus) en fonction du temps de stockage.
   On compare les valeurs de D[4,3] obtenues après différentes durées de stockage dans les différentes conditions de température, par rapport à une référence de D[4,3] mesurée sur la suspension juste après sa préparation (t = 0).
   On fixe arbitrairement les seuils de durée de stockage avec conservation de la répartition granulométrique des **PV** comme indiqué ci-après :
   * supérieure ou égale à trois mois à 37 °C ;
   * supérieure ou égale à neuf mois à température ambiante ;
   * supérieure ou égale à dix-huit mois à 4 °C.

De préférence, les **PV** de gel-composite selon l'invention possèdent au moins deux des caractéristiques fonctionnelles α₁, α₂, α₃, α₄ et, plus préférentiellement encore, α₁, α₂ et α₃.

Ces caractéristiques sont illustrées infra dans les exemples.

Comme indiqué supra, la sélection du copolyaminoacide est l'un des paramètres cruciaux de l'invention. C'est ainsi que, conformément à une caractéristique préférée, le copolyaminoacide **(III)** présente :
* des comonomères AAᵢ choisis dans le groupe d'aminoacides suivants : acide glutamique, acide aspartique, ornithine, arginine, lysine, asparagine, histidine et leurs mélanges,
* ainsi que des comonomères AAₒ choisis dans le groupe d'aminoacides suivants : leucine, tyrosine, phénylalanine, valine, cystine, isoleucine et leurs mélanges.

Plus préférentiellement encore, le copolyaminoacide **(III)** comprend un comonomère AAᵢ formé par l'acide glutamique et/ou l'acide aspartique, ainsi qu'un monomère AA₀ formé par la leucine et/ou l'isoleucine et/ou la tyrosine et/ou la phénylalanine.

Les copolyaminoacides **(III)** linéaires selon l'invention peuvent être de structure statistique ou de structure dibloc, tribloc ou multiblocs, la structure dibloc étant préférée pour des raisons de non-immunogénicité.

Par structure statistique, on entend un copolymère obtenu à partir de comonomères ayant des rapports de réactivité différents, ce qui implique que la composition de ces copolymères, dits statistiques, varie en fonction du degré de conversion.

Un exemple de copolyaminoacide **(III)** plus spécifiquement adapté à l'invention est le Leu/Glu et, en particulier, celui dosé à 50/50.

Le fait de limiter le nombre de comonomères à deux acides aminés, un AA₀ et un AAᵢ, permet de minimiser l'immunogénicité des particules **PV.** Il s'agit là d'un avantage notable de cette forme préférée de réalisation de l'invention.

Outre leur structure linéaire préférée à enchaînements α-peptidiques, les copolyaminoacides **(III)** ont, pour autre caractéristique, d'avoir une masse molaire M_{w} élevée, c'est-à-dire supérieure ou égale à 5 000 D, de préférence comprise entre 8 000 et 100 000 D. De manière plus privilégiée, on sélectionne la masse molaire M_{w} des copolyaminoacides **(III)** en fonction de leur nature : multibloc ou statistique (rondom). Dans le cas de polyaminoacides multibloc **(III),** en particulier dibloc, on préfère ceux de masse M_{w} supérieure ou égale à 5 000 D et, de préférence, comprise entre 3 000 et 100 000 D et, plus préférentiellement encore, entre 5 000 et 20 000 D. S'agissant des copolyaminoacides **(III)** de type statistiques randoms, on retient, plus volontiers, ceux de M_{w} supérieure ou égale à 50 000 D, de préférence comprise entre 10 000 et 300 000 D et, plus préférentiellement encore, comprise entre 10 000 à 100 000 D.

En fait, ces copolyaminoacides **(III)** sont des copolymères amphiphiles formés par un premier type de monomères AA₀ qui est un acide aminé neutre hydrophobe et par au moins un deuxième type de comonomères AAᵢ qui est un acide aminé présentant une chaîne latérale de fonctionnalité carboxyle (Glu/Asp) ionisable à des pH physiologiques non dénaturants pour les protéines. Ces **PAA (III)** amphiphiles peuvent interagir, à la fois avec des substances hydrophobes et avec des substances hydrophiles, ce qui leur confère des propriétés remarquables comme tensioactifs ou dispersants. Ils participent ainsi à l'effet étonnant par lequel les **PV** acquièrent et conservent la qualité de corps certains stables.

S'agissant de la disponibilité des **PA (III),** il faut signaler qu'il existe de nombreuses techniques de synthèse de polymères d'α-aminoacides à blocs ou statistiques ou de polymères à chaînes multiples ou bien encore de polymères contenant une séquence déterminée d'aminoacides (cf. Encyclopedia of Polymers Science and Engeneery (vol. 12, page 786, Ivan WILEY & Sons). De nombreux dérivés d'aminoacides et de peptides ont été utilisés comme monomères pour la préparation des polyaminoacides. Cependant, les monomères servant le plus couramment sont les anhydrides des N-carboxy-α-aminoacides dont la préparation est donnée, par exemple, dans Biopolymers, 15, 1869 (1976). Les techniques de polymérisation de ces monomères sont connues de l'homme de l'art et sont détaillées dans l'ouvrage de H. R. KRICHELDORF "α-Aminoacid-N-Carboxy Anhydrides and Related Heterocycles" Springer Verlag (1987); Les techniques de synthèse impliquent, généralement, de protéger les fonctions réactives des acides aminés à chaînes latérales ionisables, afin qu'elles n'interfèrent pas lors de l'étape de polymérisation. Il s'ensuit qu'une étape de déprotection est nécessaire pour rétablir la fonctionnalité des chaînes latérales ionisables du polymère. On peut citer, par exemple, les procédés de déprotection par saponification des esters méthyliques (STAHMAN et coll ; J. Biol. Chem., 197, 771 (1952) ; KYOWA HAKKO, FR 2 152 582) ou de débenzylation [BLOUT et coll. ; J. Amer. Chem. Soc., 80, 4631 (1858)].

S'agissant désormais de l'huile **(I),** on peut indiquer qu'au sens de l'invention une huile désigne un corps liquide à température ambiante et qui, en outre, n'est pas ou peu miscible à l'eau. Les matières grasses liquides à température ambiante répondent à cette définition. Il en va de même en ce qui concerne les huiles silicones.

Aussi, conformément à une caractéristique préférée de l'invention, l'huile **(I)** est formée par un ou plusieurs composés gras sélectionnés dans le groupe suivant
* triglycéride(s) d'acide d'ester gras à chaînes moyennes d'origine animale, végétale ou synthétique,
* paraffine(s),
* huile(s) polysiloxane(s),
* acides gras d'origine animale ou végétale,
* acides gras, leurs esters et/ou leurs sels.

Comme exemple d'huile particulièrement adaptée au **PV** selon l'invention, on peut citer
- les huiles triglycéridiques du type huile de noix de coco (telle que celle commercialisée sous la marque "MYGLIOL®" par la Société DYNAMIT NOBEL,
- l'huile de maïs,
- la paraffine,
- l'huile d'olive,
- le palmitate d'isopropyl,
- le stéarate de butyle,
- l'oléate d'éthyle,
- les esters d'acides eicosapentanoïques docosahexanoïques,
- le benzoate d'alkyle,
- les acides eicosapentanoïques docosahexanoïques,
- les triglycérides d'acides eicosapentanoïque docosahexanoïque,
- les triglicérides d'acides caprylique,
- l'huile de silicone.

L'huile **(I)** peut être constituée par un seul type de corps gras liquide à température ambiante ou par un mélange de plusieurs de ceux-ci.

Selon une variante, l'huile ou les huiles **(I)** peuvent être fractionnées ou non. Le fractionnement permet d'éliminer certaines coupes d'acides gras, de façon à modifier la température de fusion et la viscosité.

S'agissant précisément de la température de fusion, on peut préciser, pour fixer les idées, que celle de l'huile **(I)** est inférieure ou égale à 50 °C, de préférence à 40 °C et, plus préférentiellement, à 35 °C.

La viscosité de l'huile **(I)** à 25 °C est, en pratique, comprise entre 10 mPa.s et 3 000 mPa.s.

Sur le plan quantitatif, l'huile **(I)** représente de 9 à 90 % en poids, de préférence de 20 à 80 % en poids et, plus préférentiellement encore, de 40 à 60 % en poids des **PV.**

En ce qui concerne la phase aqueuse **(II)** des microparticules lipidiques ici considérées, elle est, de préférence, constituée par une solution saline qui sera, avantageusement, tamponnée, de manière à avoir un pH compris entre 5 et 9, de préférence entre 6 et 8 et, plus préférentiellement encore, de l'ordre de 7,4.

Les solutés de cette solution sont des sels comme, par exemple, le NaCl.

Avantageusement, la molarité de la solution saline est comprise entre 10⁻⁴ M et 1 M, de préférence entre 10⁻² M et 0,5 M environ.

Les tampons éventuellement mis en oeuvre sont, par exemple, les suivants : phosphate, phtalate, borate, etc.

Le fait que la phase aqueuse soit préférentiellement formée par une solution saline, n'est pas exclusive de la variante dans laquelle on a affaire à de l'eau, avantageusement désionisée.

Les microparticules lipidiques préférées conformément à l'invention sont celles caractérisées par la composition suivante :
**(I)** triglycéride(s) d'acide d'ester gras à chaînes moyennes, l'huile de coco étant particulièrement préférée,
**(II)** eau déionisée ou solution saline tamponnée, le pH de cette phase aqueuse étant compris entre 6 et 8,
**(III)** copolyaminoacide de type Leu/Glu, de préférence 50/50.

Au-delà des caractéristiques structurelles et fonctionnelles des PV, telles qu'elles ont été énoncées ci-dessus, la présente invention a également pour objet un procédé de préparation de microparticules, notamment du type de celles définies supra, caractérisé en ce qu'il comprend, essentiellement, les étapes suivantes, successives ou non :
- **a -** préparation d'un gel à partir de la phase aqueuse **(II)** et d'au moins un copolyaminoacide **(III), (II)** et **(III)** étant tels que définis dans la description,
- **b -** mise en présence du gel issu de l'étape **- a -** avec de l'huile **(I)**, telle que définie dans la description,
- **c -** agitation du mélange gel + **(I)** conduisant à une dispersion de microparticules dans une phase continue huileuse ou aqueuse **(I),**
- **d -** éventuelle séparation des microparticules et de la phase continue huileuse ou aqueuse, de préférence par centrifugation,
- **e -** éventuelle redispersion des microparticules recueillies à l'issue de l'étape **- e -** dans un liquide de stockage,
- **f -** éventuel traitement de lyophilisation des microparticules de l'étape **- d -**, redispersées ou non.

L'un des fondements de ce procédé et des microparticules qui en découlent tient à l'étape - **a** - de préparation d'un gel issu de l'association du copolyaminoacide **(III)** et de la phase aqueuse **(II).** Il s'agit, plus précisément, de mélanger **(II)** à **(III)** dans des proportions telles que **(III)** représente de 2 à 50 %, de préférence de 5 à 30 % en poids du gel **(III)** + **(II).** Ce mélange s'effectue sous agitation grâce à tout moyen d'agitation connu en lui-même et approprié. Il peut s'agir, par exemple, d'un dispositif du type Vortex ou bien encore du type rotor-stator, barreau magnétique, ultrasons, homogénéiseur haute pression.
Avantageusement, cette étape **- a -** est assortie d'un traitement d'élimination de la mousse formée lors de l'agitation. Une telle élimination peut être, par exemple, réalisée par centrifugation.
Le gel obtenu à l'étape **- a -** est ensuite mis en présence de tout ou partie de l'huile **(I)** (étape **- b -**), le mélange ainsi formé étant ensuite soumis à une agitation (étape **- c -**) avec des moyens d'agitation du même type que ceux évoqués pour l'étape **- a -**. Cette étape **- c -** correspond à l'étape de formation des microparticules lipidiques.
L'introduction de l'huile **(I)** dans le mélange de l'étape **- b -** peut être effectuée en une ou plusieurs fois au cours de l'étape **- c -**.
Il est clair que les conditions d'agitation sont déterminantes pour la formation des **PV** lors de l'étape **- c -**, en particulier eu égard à la nature de la phase homogène contenant les **PV** : eau ou huile.

Aussi, selon un mode préféré de mise en oeuvre de l'invention, conduisant à des **PV** gel-composite en suspension dans une phase aqueuse, cette agitation **- c -** est réalisée à l'aide d'un dispositif rotor/stator et en mettant en oeuvre une vitesse d'agitation comprise entre 1 000 et 40 000, de préférence 5 000 et 25 000 tr/min.

Selon une variante de ce mode préféré, dans laquelle on vise l'obtention de **PV** en suspension dans une phase huileuse, on enrichit la suspension en huile.
Au terme de l'étape **- c -**, il est possible d'obtenir un produit intermédiaire intéressant, formé par une dispersion concentrée de **PV** dans l'eau ou une suspension de **PV** dans l'huile. Dans ce dernier cas, on peut éliminer l'huile excédentaire et on obtient alors un sédimentat concentré en **PV.**

La présente invention a donc également pour objet cette dispersion et ce sédimentat concentrés de **PV** dans l'eau, qui peuvent être considérés comme des produits intermédiaires.

Ces produits intermédiaires sont caractérisés par :
* une concentration moyenne en copolyaminoacides **(III)** variant de 1 % à 50 % en poids, de préférence de 2 % à 40 % en poids, cette concentration s'établissant, plus préférentiellement encore, de 3 % à 30 % en poids ;
* une concentration en huile **(I)** représentant de 9 à 90 % en poids, de préférence de 20 à 80 % en poids et, plus préférentiellement encore, de 40 à 60 % en poids ;
* une concentration en phase aqueuse de 5 à 90 %, de préférence de 10 à 70 %, plus préférentiellement encore de 30 à 50 % en poids ;
les concentrations ci-dessus étant exprimées par rapport à la masse totale de la dispersion ou du sédimentat concentrés en **PV.**

Les quantités relatives d'huile **(I)** et de phase aqueuse **(II)** prévaudront l'une sur l'autre, selon la nature hydrophile ou hydrophobe visée pour les **PV.** Dans l'application biovecteur, cette nature sera fonction du principe actif à vectoriser.

Pour récapituler, on peut donc indiquer que les microparticules selon l'invention peuvent être définies par les caractéristiques quantitatives suivantes, selon lesquelles leurs constituants **(I), (II), (III)** sont présents dans les proportions ci-après, exprimées en % en poids par rapport à **(I)** + **(II)** + **(III)** :
**(I)** 9 à 90, de préférence 20 à 80 et, plus préférentiellement encore, 40 à 60,
**(II)** 5 à 90, de préférence 10 à 70 et, plus préférentiellement encore, 30 à 50,
**(III)** 1 à 50, de préférence 2 à 20 et, plus préférentiellement encore, 3 à 10.
La récupération des microparticules (étape **- d -**) peut être effectuée par tous moyens connus et appropriés, la centrifugation ou la décantation sont des exemples de tels moyens.
Le milieu de dispersion des **PV** récupérées à l'étape **- d -** peut être de l'eau déionisée filtrée ou une solution solide tamponnée, éventuellement additionnée d'au moins un agent bactériostatique.

Avantageusement, la température à laquelle s'effectuent les étapes **- a -** à **- c -**, voire - **d - à - f -** du procédé selon l'invention, est comprise entre 4 °C et 60 °C. La température ambiante est particulièrement appropriée, celle-ci étant, de préférence, comprise entre 10 et 35 °C.

Comme cela ressort de ce qui précède, le procédé selon l'invention permet la génération spontanée de microparticules lipidiques grâce à un protocole remarquable de simplicité, d'économie et donc de faisabilité industrielle. On observera, également, que le procédé selon l'invention est incontestablement et invariablement sûr, puisqu'il ne prévoit pas le recours à des réactifs ou solvants toxiques.

Les paramètres importants du procédé selon l'invention sont, notamment, la nature de l'huile **(I)**, la composition du copolyaminoacide **(III)** et sa concentration, la concentration de la solution saline **(II),** les conditions d'agitation et le pH du milieu réactionnel.

L'homme du métier est apte à maîtriser relativement aisément toutes ces conditions de préparation des **PV** selon l'invention. En particulier, il est tout à fait à sa portée de contrôler et d'ajuster la granulométrie des microparticules lipidiques en jouant sur les conditions d'agitation et sur le rapport des viscosités des phases en présence, entre autres.

Dans la mesure où l'une des applications les plus remarquables des particules selon l'invention est le transport sous protection de principes actifs **(PA)** dans l'organisme humain ou animal, il convient d'apporter des précisions, à ce stade de l'exposé, sur la ou les techniques d'inclusions du **PA** dans les **PV.**

L'une des techniques d'inclusion préférée conformément à l'invention consiste à dissoudre ou à mettre en suspension ledit **PA**
- dans l'huile **(I)**,
- et/ou dans la phase aqueuse **(II),**
- et/ou dans le polyaminoacide **(III),**
- et/ou dans le gel de l'étape **- a -**.

Le **PA** à inclure dans les **PV** peut se présenter sous forme solide ou sous forme de solution ou de dispersion.

Le principe actif, susceptible d'être inclus ou incorporé dans les **PV** selon l'invention, peut être médicamenteux et/ou nutritionnel. Dans le cas où il est médicamenteux, le **PA** est, de préférence, choisi parmi :
* les protéines et/ou les peptides parmi lesquels les plus préférentiellement retenus sont : les hémoglobines, les cytochromes, les albumines, les interférons, les antigènes, les anticorps, la calatonine, l'érythropoïétine, l'insuline, les hormones de croissance, le facteur IX, l'interleukine ou leurs mélanges,
* les polysaccharides, l'héparine étant plus particulièrement sélectionnée,
* les acides nucléiques et, préférablement, les oligonucléotides d'ARN et/ou d'ADN,
* et leurs mélanges.

Les **PA,** que l'on peut classer dans la catégorie des médicaments et qui sont propres à être vectorisés par les particules selon l'invention, sont les vaccins.

A titre d'exemples de **PA** nutritionnels, on peut citer les vitamines, les acides aminés et les oligo-éléments.

### APPLICATION INDUSTRIELLE :

Selon un autre de ses aspects, l'invention vise également l'utilisation de **PV** chargés en **PA,** pour la fabrication de médicaments, notamment du type système à libération contrôlée de **PA.**

La présente invention concerne enfin les médicaments et les spécialités pharmaceutiques et nutritionnelles comprenant les microparticules chargées en **PA,** telles que décrites ci-dessus.

Les spécialités pharmaceutiques concernées sont, notamment, celles pour administration, de préférence, par voies orale, nasale, vaginale, oculaire, sous-cutanée, intraveineuse, intramusculaire, intradermique, intrapéritonéale, intracérébrale ou parentérale.

Les applications de l'invention ne sont pas limitées à la vectorisation, au transport de **PA** de nature médicamenteuse ou nutritionnelle. En effet, il est tout à fait concevable que le **PA,** susceptible d'être inclus ou incorporé dans les **PV,** soit un produit cosmétique ou phytosanitaire.

Les applications cosmétiques envisageables sont, par exemple, les compositions applicables par voie transdermique.

Les produits phytosanitaires concernés peuvent être, par exemple, des herbicides et/ou des fongicides et/ou des bactéricides et/ou des virucides et/ou des insecticides, entre autres.

La présente invention a également pour objet les compositions phytosanitaires et cosmétiques comprenant des **PV** chargées en **PA** du type de ceux visés supra.

Les exemples qui suivent permettront de mieux comprendre l'invention dans ses différents aspects produits/procédés/applications. Ces exemples illustrent la préparation de microparticules **PV,** chargées ou non en principes actifs et à base d'huile **(I),** d'eau **(II)** et de **PAA (III).** Ces exemples montrent, également, les caractéristiques de structure, ainsi que les propriétés desdites microparticules.

L'illustration des exemples est donnée par les **fig. 1** à **11** dont la description suit.

### DESCRIPTION DES FIGURES

**La fig. 1** est une photographie en microscopie électronique à balayage (MEB) des **PV** gel-composite préparées à l'exemple 4, grossissement : 1 500 x.

**La fig. 2** est une photographie en microscopie électronique à balayage (MEB) des **PV** gel-composite préparées à l'exemple 5, grossissement : 3 200 x.

La **fig. 3** est un histogramme de répartition volumique de **PV** de gel-composite : volume différentiel (V) en % en fonction du diamètre (D) en µm (exemple 4).

La **fig. 4** est un histogramme de répartition volumique de **PV** de gel-composite lyophilisées et réhydratées : volume différentiel (V) en % en fonction du diamètre (D) en µm (exemple 4).

La **fig. 5** est un histogramme de répartition volumique de **PV** de gel-composite : volume différentiel (V) en % en fonction du diamètre (D) en µm (exemple 5).

La **fig. 6** est un histogramme de répartition volumique de **PV** de gel-composite : volume différentiel (V) en % en fonction du diamètre (D) en µm (exemple 6).

La **fig. 7** est un histogramme de répartition volumique de **PV** de gel-composite (volume différentiel (V) en % en fonction du diamètre (D) en µm) :
- courbe 1 : avant lyophilisation/réhydratation,
- courbe 2 : après lyophilisation/réhydratation (exemple 7).

La **fig. 8** représente un histogramme donnant les diamètres moyens volumiques D[4,3] et les écarts-types (SD) mesurés pour les répartitions granulométriques des **PV** selon les essais 1 à 14 de l'exemple 9.

La **fig. 9** est un graphe donnant le pourcentage R de relargage du **PA** = cytochrome en fonction du temps de séjour T en heures (exemple 11).

### EXEMPLES

### EXEMPLE 1 : SYNTHESE DU RANDOM (STATISTIQUE) POLY(LEUCINE-GLUTAMATE DE SODIUM) 50/50.

### ETAPE 1) : COPOLYMERISATION DES NCA- LEU ET NCA-GLU(OME) : POLY(LEU-CO-GLU(OME)) 50/50 :

Dans un réacteur de 1 l, muni d'un agitateur en verre, d'une arrivée d'azote et d'une sortie reliée à un bulleur, on introduit, sous courant d'azote, 15,0 g de N-carboxyanhydride de la glutamate de méthyle (NCA-Glu(OMe) : 0,08 mole) et 12,5 g de N-carboxyanhydride de la leucine (NCA-Leu : 0,08 mole). 381 ml de dioxane sont rajoutés et le milieu réactionnel est porté à 40 °C.
Après dissolution des NCA, on introduit 24 ml d'eau, suivi de 0,22 ml de triéthylamine (soit 1 % molaire par rapport aux NCA). Le suivi de la polymérisation est effectué par IR en observant la disparition des bandes carbonyles à 1 860 et 1 790 cm⁻¹. La durée de polymérisation varie entre 1,5 h et 3 h selon la composition des monomères. Après disparition totale des bandes, le milieu réactionnel est dilué par 380 ml de dioxane, puis homogénéisé pendant 3 h à température ambiante. Le copolymère est récupéré par précipitation dans 5 l d'eau sous bonne agitation. Le produit est filtré et séché à 50 °C sous vide pendant 12 h.
La masse de copolymère obtenu est de 18,4 g, soit un rendement pondéral de 90 %.
RMN 1H (acide trifluoroacétique-d) : 0,85 ppm (CH₃-Leu, 6H*0,5) ; 1,58 (CH₂ et CHMe₂ Leu, 3H*0,5); 2,10 et 2,22 (CH₂-Glu, 2H*0,5) ; 2,58 (CH₂-Glu ; 2H*0,5) ; 3,75 (CH₃-Glu, 3H*0,5) ; 4,62 (NCHCO-Leu, 1H*0,5) ; 4,70 (NCHCO-Glu, 1H*0,5). Viscosité réduite (0,5 g/dl dans l'acide trifluoroacétique) à 25 °C = 2,2 dl/g.

### ETAPE 2) : HYDROLYSE DE L'ESTER METHYLIQUE DU POLY(LEU-CO-GLU(OME)) 50/50 :

Le copolymère obtenu précédemment (17,7 g) est placé dans un réacteur dans lequel on rajoute 354 ml d'acide trifluoroacétique. Le milieu réactionnel est porté à 40 °C sous agitation. Lorsque le copolymère est totalement dissous, on rajoute 354 ml d'eau par petites quantités. Le milieu réactionnel est maintenu sous agitation pendant 48 h.
Le polymère est récupéré par précipitation dans 5 l d'eau. Après filtration, il est à nouveau mis en suspension et agité dans l'eau pendant 0,5 h, puis filtré et essoré. La purification s'effectue par dialyse dans l'eau.
Rendement 15,9g (95 %). RMN 1H (acide trifluoroacétique-d) : identique aux polymères de départ à une exception, le signal à 3,75 (CH₃-Glu) est fortement diminué ou absent. Dans le cas présent, le taux d'esters résiduels est inférieur à 1 % par rapport aux monomères glutamate. Viscosité réduite (0,5 g/dl dans l'acide trifluoroacétique) à 25 °C = 0,95 dl/g.

### EXEMPLE 2 : SYNTHESE DU DIBLOC POLY(LEU-B-GLU(ONA)) 50/50.

Dans un réacteur de 1 l, on introduit sous agitation 15,0 g de NCA-Glu(OMe) (0,08 mole) et 180 ml de dioxane. Après dissolution, on rajoute 180 ml de toluène et le milieu est porté à 60 °C. On effectue le spectre IR de la solution avant de rajouter 0,156 g de benzylamine (1,58 % molaire/NCA). Le milieu réactionnel se trouble rapidement et, au bout de 40 minutes, les bandes caractéristiques à 1 860 et 1 790 cm⁻¹ ont disparu.
Après une heure, on introduit une solution de 12,5 g de NCA-Leu (0,08 mole) dans un mélange dioxane/toluène (15 ml de chaque). L'agitation est poursuivie pendant 18 h (cette durée n'a pas été optimisée). Les bandes carbonyles ont alors disparu.
On ajoute 100 ml de dioxane et le milieu réactionnel est homogénéisé pendant 1 h. Le copolymère est précipité dans 3 1 d'éthanol absolu sous forte agitation. Il est lavé avec 1 l d'éthanol, filtré, essoré et enfin séché à 50 °C sous vide pendant une nuit.
La masse de produit récupérée est de 19,5 g (rendement = 95 %). RMN 1H (acide trifluoroacétique-d): 0,85 ppm (CH₃-Leu, 6H*0,5) ; 1,58 (CH₂ et CHMe₂ Leu, 3H*0,5) ; 2,10 et 2,22 (CH₂-Glu, 2H*0,5) ; 2.58 (CH₂-Glu ; 2H*0,5) ; 3,75 (CH₃-Glu, 3H*0,5) ; 4,62 (NCHCO-Leu, 1H*0,5) ; 4,70 (NCHCO-Glu, 1H*0,5). Viscosité réduite (0,5 g/dl dans l'acide trifluoroacétique) à 25 °C = 0,62 dl/g.
La deuxième étape d'hydrolyse des esters méthyliques est identique à celle décrite dans l'exemple 1, étape 2. Rendement 95 %. RMN 1H (acide trifluoroacétique-d): identique aux polymères de départ à une exception, le signal à 3,75 (CH₃-Glu) est fortement diminué ou absent. Dans le cas présent, le taux d'esters résiduels est inférieur à 1 % par rapport aux monomères glutamate. Viscosité réduite (0,5 g/dl dans l'acide trifluoroacétique) à 25 °C = 0,55 dl/g.

### EXEMPLE 3 : SYNTHESE DU TRIBLOC POLY(GLU(ONA)-LEU-GLU(ONA)) 25/50/12.

Dans un réacteur de 1 l, on introduit sous agitation 7,5 g de NCA-Glu(OMe) (0,04 mole) et 180 ml de dioxane. Après dissolution, on rajoute 180 ml de toluène et le milieu est porté à 60 °C. On effectue le spectre IR de la solution avant de rajouter 0,156 g de benzylamine.
Après la disparition totale du monomère, on introduit une solution de 12,5 g de NCA-Leu (0,08 mole) dans un mélange dioxane/toluène (15 ml de chaque). L'agitation est poursuivie pendant 18 h. Ensuite, on introduit à nouveau 7,5 g de NCA-Glu(OMe) (0,04 mole) qu'on permet de réagir pendant 12 heures. On ajoute 100 ml de dioxane et le milieu réactionnel est homogénéisé pendant 1 h.
Le copolymère est précipité dans 3 1 d'éthanol absolu sous forte agitation. Il est lavé avec 1 l d'éthanol, filtré, essoré et enfin séché à 50 °C sous vide pendant une nuit.
La masse de produit récupéré est de 19,4 g (rendement = 95 %). RMN 1H (acide trifluoroacétique-d) : 0,85 ppm (CH₃-Leu, 6H*0,5) ; 1,58 (CH₂ et CHMe₂ Leu, 3H*0,5) ; 2,10 et 2,22 (CH₂-Glu, 2H*0,37) ; 2,58 (CH₂-Glu; 2H*0,37) ; 3,75 (CH₃-Glu, 3H*0,37) ; 4,62 (NCHCO-Leu, 1H*0,5) ; 4,70 (NCHCO-Glu, 1H*0,37). Viscosité réduite (0,5 g/dl dans l'acide trifluoroacétique) à 25 °C = 0,58 dl/g.

La deuxième étape d'hydrolyse des esters méthyliques est identique à celle décrite dans l'exemple 1, étape 2. RMN 1H (acide trifluoroacétique-d) : identique aux polymère de départ à une exception, le signal à 3,75 (CH₃-Glu) est fortement diminué ou absent. Dans le cas présent, le taux d'esters résiduels est inférieur à 1 % par rapport aux monomères glutamate. Viscosité réduite (0,5 g/dl dans l'acide trifluoroacétique) à 25 °C = 0,38 dl/g.

### EXEMPLE 4 : MICROPARTICULES NEUTRES PREPAREES A PARTIR DE MIGLYOL®, D'EAU ET POLY LEUCINE-CO-GLUTAMATE DE SODIUM DE COMPOSITION 50/50 ET DE STRUCTURE RANDOM (STATISTIQUE).

### 4.1- METHODOLOGIE :

### 4.1.1 - ETAPE a): PREPARATION DU GEL :

- On introduit, dans un tube à hémolyse, 50 mg de lyophilisat de polyleucine-co-glutamate de sodium (noté Leu/Glu), de composition 50/50, de structure statistique, synthétisé selon l'exemple 1 et de M_{w} de 110 000.
- On ajoute la phase aqueuse, typiquement 500 mg, qui peut être composée par de l'eau désionisée ou une solution saline tamponnée, par exemple du PBS 0,01 M (tampon phosphate, pH 7,4 à 25 °C).
- On laisse le lyophilisat de polyaminoacide s'hydrater pendant 2 heures à température ambiante.
- Le gel obtenu se présente sous la forme d'une solution colloïdale visqueuse diffusant dans le bleu.

### 4.1.2 - ETAPES b) à e) : PREPARATION DES MICROPARTICULES :

(i) Procédé pour obtenir des **PV** dans une phase continue huileuse :
   - On introduit, dans le tube à hémolyse contenant le gel de polyaminoacide, 2 ml de la phase lipidique qui est composée d'huile de coco fractionnée, connue sous le nom commercial de Miglyol® (DYNAMIT NOBEL).
   - On agite le mélange avec un homogénéisateur du type rotor/stator (ULTRA-TURRAX T8, TKA LABORTECHNIK). On obtient une dispersion d'aspect laiteux de microparticules dans une phase continue lipidique.
   - Le tube à hémolyse, contenant la dispersion de microparticules, est centrifugé. La phase surnageante, composée de Miglyol® excédentaire, est séparée du sédimentat par simple écoulement On recueille ainsi 1 340 mg d'huile.
   - Le sédimentat est redispersé dans de l'eau désionisée, contenant un bactériostatique : le Thimérosal® (50 µg/ml), ou dans une solution saline tamponnée, par exemple du PBS 0,01 M (tampon phosphate, pH 7,4 à 25 °C) contenant du Thimérosal® (50 µg/ml). Le volume total de la dispersion obtenue est de 5 000 µl.
(ii)Procédé pour obtenir des **PV** dans une phase continue aqueuse :
   - On introduit, dans le tube à hémolyse contenant le gel de polyaminoacide, 400 ± 2 µl de la phase lipidique qui est composée d'huile de coco fractionnée connue sous le nom commercial de Miglyol® (DYNAMIT NOBEL).
   - On agite le mélange avec un homogénéisateur du type rotor/stator (ULTRA-TURRAX T8, IKA LABORTECHNIK). On obtient une dispersion très dense laiteuse de microparticules dans une phase continue eau.
   - La dispersion est alors diluée dans de d'eau désionisée contenant du Thimérosal® (50 µg/ml), ou dans une solution saline tamponnée, par exemple du PBS 0,01 M (tampon phosphate, pH 7,4 à 25 °C) contenant du Thimérosal® (50 µg/ml). Le volume total de la dispersion obtenue est de 5 000 µl.

### 4.2 - CARACTERISATION DES MICROPARTICULES :

• La composition globale des microparticules avant redispersion est :
* 4 % en polyaminoacide,
* 53 % en Miglyol®,
* 43 % d'eau ou de solution saline.

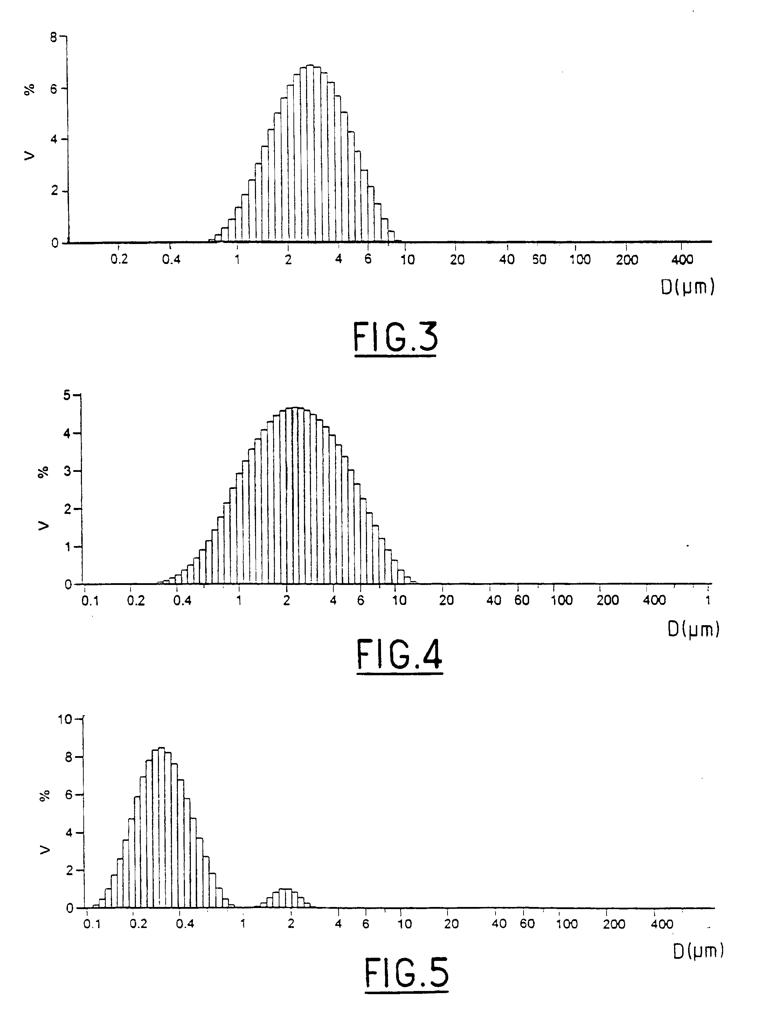

• La répartition granulométrique des microparticules est mesurée par diffraction laser. L'appareil utilisé est un COULTER LS 130. Le modèle de calcul choisi est le modèle de FRAUNHOFER avec "PIDS". L'histogramme de répartition volumique est donné en **fig.** 3. Le profil est monomodal, le diamètre moyen de référence **D[4,3] est de 2,8 µm** avec un écart type (SD) de 1,1 µm.
Exprimée différemment, la répartition granulométrique est la suivante :

| | | | | | |
|---|---|---|---|---|---|
| % de PV : | 10,00 | 25,00 | 50,00 | 75,00 | 90,00 |
| Taille en µm inférieure à : | 5,165 | 3,876 | 2,730 | 1,901 | 1,394 |

• Les microparticules ont été observées en microscopie électronique à balayage (MEB) en platine froide. Les photographies sont données **fig. 1.**
• Les microparticules conservent leur identité et leur intégrité dans les solvants, tels que l'acétone, le diméthylsulfoxyde, l'éthanol et dans des milieux aqueux dans la gamme de pH comprise entre pH = 2 et pH = 13.
• ***Test α***_{***1***} ***:*** *Lyophilisation/réhydratation :*
La suspension, diluée dans 50 ml, est lyophilisée pendant 48 h. On récupère 529 mg de lyophilisât de microparticules. La réhydratation de la totalité du lyophilisat dans 5 ml de PBS conduit, spontanément, à une suspension de microparticules présentant un profil granulométrique, monomodal, similaire à celui des microparticules avant lyophilisation (cf. **fig. 4).** Le diamètre moyen volumique, D[4,3], est de 2,9 µm et l'écart type de 2 µm, soit un Δ de 3,5 % par rapport au D[4,3] de référence.
***Test* α**_{**1**} ***:*** *résultats :*

| | | | | | |
|---|---|---|---|---|---|
| % de PV : | 10 | 25 | 50 | 75 | 90 |
| Taille en µm inférieure à : | 0,907 | 1,385 | 2,322 | 3,873 | 5,816 |

• ***Test α***_{***2***} ***:***
On trouve un D[4,3] de 3 µm avec une estimation de l'écart type de 2 µm, soit un Δ de 7,1 % par rapport au D[4,3] de référence.
• ***Test α***_{***3***} ***:***
* à pH 2, on mesure un D[4,3] de 3,9 µm avec un SD de 2,9 µm, soit un Δ de 3,5 % par rapport au D[4,3] de référence,
* à pH 13, on mesure un D[4,3] de 3,0 µm avec un SD de 2,2 µm, soit un Δ de 7,1 % par rapport au D[4,3] de référence.

• ***Test α***_{***4***} ***:***
La stabilité de forme des microparticules stockées en dispersion aqueuse ou bien saline, à 4 °C, 37 °C et à température ambiante, est analysée par un suivi de la granulométrie des microparticules. On considère les microparticules physiquement stables lorsque la modification enregistrée du diamètre moyen D[4,3] n'excède pas plus de 20 % du D[4,3] initial.
***Test α***_{***4***} ***:*** résultats :

| | | | |
|---|---|---|---|
| Température de stockage | 4 °C | T ambiante | 37 °C |
| Stab. en dispersion aqueuse | | > 1 an | |
| Stab. en dispersion saline | > 6 mois | | > 3 mois |

### EXEMPLE 5 : NANOPARTICULES NEUTRES PREPAREES A PARTIR DE MIGLYOL®, D'EAU ET DE POLY LEUCINE-CO-GLUTAMATE DE SODIUM DE COMPOSITION 50/50 ET DE STRUCTURE RANDOM.

### 5.1- METHODOLOGIE :

### 5.1.1 - ETAPE a) : PREPARATION DU GEL :

- On procède selon l'exemple 4.1.1 en mettant en oeuvre une masse de 150 mg du polyaminoacide cité précédemment. La phase aqueuse, est alors typiquement de 1 350 mg.

### 5.1.2 - ETAPE b) à e) : PREPARATION DE NANOPARTICULES :

- On introduit dans le tube à essai contenant le gel de polyaminoacide 1 200µl de Miglyol®.
- On agite le mélange avec un homogénéisateur du type rotor/stator (ULTRA-TURRAX T8, IKA LABORTECHNIK). On obtient ainsi une suspension aqueuse concentrée et visqueuse de microcapsules.
- On dilue cette suspension dans un volume total de 15 ml d'eau désionisée ou de solution isotonique tamponnée phosphate à pH 7,4.
- On introduit la suspension diluée de microparticules dans le réservoir d'un homogénéiseur haute pression de type Microfluidizer M-110S de marque MICROFLUIDICS.
- On pratique une homogénéisation de la suspension diluée de microparticules pendant 10 min, en appliquant une pression d'entrée sur la pompe de l'ordre de 5 bars.
- On recueille alors, par vidange du réservoir, une suspension de nanoparticules.

### 5.2 - CARACTERISATION DES NANOPARTICULES :

• La composition globale des nanoparticules avant dilution est : 6 % en polyaminoacide ; 43 % en Miglyol® ; 51 % d'eau ou de solution saline.
• La répartition granulométrique des microparticules est mesurée par diffraction laser. L'appareil utilisé est un COULTER LS130 ; le modèle de calcul choisie est le modèle de Psl O.M.D inclued "PIDS". L'histogramme de répartition volumique est donné en **fig. 5.** Le diamètre moyen de référence **D[4.3] est de 0,420 µm** avec un écart type de 0,37 µm.

| | | | | | |
|---|---|---|---|---|---|
| % de PV: | 10 | 25 | 50 | 75 | 90 |
| Taille en µm inférieure à : | 0,191 | 0,242 | 0,321 | 0,434 | 0,592 |

• ***Test α***_{***2***} ***:***
On obtient un D[4,3] de 0,45 µm et un SD de 0,41 µm, soit un Δ de 7,1 % par rapport au D[4,3] de référence.

### EXEMPLE 6 : MICROPARTICULES NEUTRES PREPAREES A PARTIR DE MIGLYOL®, D'EAU ET POLY LEUCINE-CO-GLUTAMATE DE SODIUM DE COMPOSITION 50/50 ET DE STRUCTURE DIBLOC.

### 6.1 - METHODOLOGIE :

### 6.1.1 - ETAPE α) : PREPARATION DU GEL :

- On introduit, dans un tube à hémolyse, 146 mg de lyophilisat de poly leucine-co-glutamate de sodium (noté LEU/GLU) synthétisé selon l'exemple 2.
- On ajoute la phase aqueuse, typiquement 702 ± 0,2 mg, qui peut être composée par de l'eau désionisée filtrée à 0,22 µm, ou une solution saline tamponnée, par exemple du PBS 0,01 M (Solution isotonique, tamponnée phosphate à pH 7,4 à 25 °C ).
- On laisse le lyophilisat de polyaminoacide s'hydrater.
- Le gel obtenu se présente sous la forme d'une solution colloïdale visqueuse diffusant fortement dans le blanc.

### 6.1.2 - ETAPES b) à e) : PREPARATION DES MICROCAPSULES :

- On procède ensuite comme dans l'exemple 4.1.2(i) Après centrifugation, on recueille 1 518 mg d'huile.

### 6.2 - CARACTERISATION DES MICROCAPSULES :

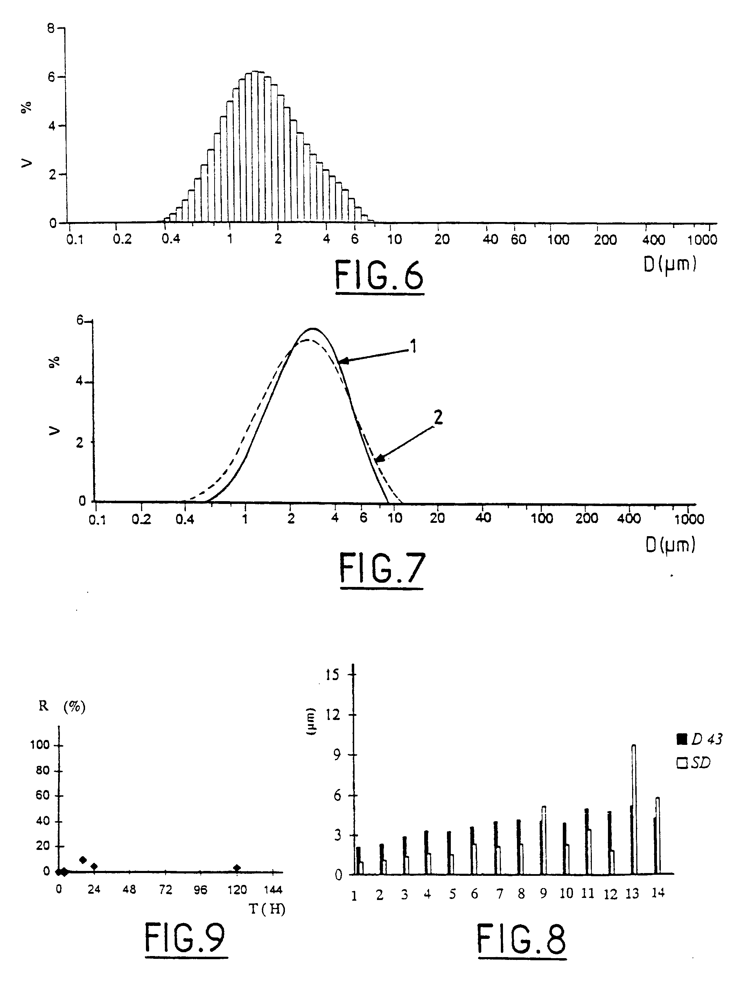

• La composition globale des microparticules avant redispersion est : 12 % en polyaminoacide ; 31 % en Miglyol® ; 57 % d'eau ou de solution saline.
• La répartition granulométrique des microparticules est mesurée par diffraction laser. L'appareil utilisé est un COULTER LS130 ; le modèle de calcul choisi est le modèle de FRAUNHOFER inclued "PIDS". L'histogramme de répartition volumique est donné en **fig. 6.** Le profil est monomodale, le diamètre moyen de référence **D[4,3] est de 1,9 µm** avec un écart type de 1,2 µm.

| | | | | | |
|---|---|---|---|---|---|
| % de PV : | 10 | 25 | 50 | 75 | 90 |
| Taille en µm inférieure à : | 0,801 | 1,096 | 1,605 | 2,433 | 3,678 |

• Les microparticules ont été observées en MEB en platine froide. Les photographies sont données en **fig. 2.**
• ***Test α***_{***4***} ***:***
On obtient un Δ de D[4,3] inférieur ou égal à 10 % par rapport au D[4,3] de référence.
***Test α***_{***4***} *- Résultats :*

| | | | |
|---|---|---|---|
| Température de stockage | 4 °C | T ambiante | 37 °C |
| Stabilité en dispersion saline | ≥ 18 mois | ≥ 12 mois | ≥ 3 mois |

### EXEMPLE 7 : MICROPARTICULES NEUTRES PREPAREES A PARTIR DE MIGLYOL®, D'EAU ET POLY LEUCINE-CO-GLUTAMATE DE SODIUM DE COMPOSITION 30/70 ET DE STRUCTURE RANDOM.

### 7.1 - METHODOLOGIE :

### 7.1.1 - ETAPE a) : PREPARATION DU GEL :

- On introduit, dans un tube à hémolyse, 100 mg de lyophilisat de poly leucine-co-glutamate de sodium (noté LEU/GLU) de composition 30/70, synthétisé selon le protocole donné à l'exemple 1 en modifiant le ratio des monomères.
- On ajoute la phase aqueuse, typiquement 400 mg, de PBS 0,01 M (solution isotonique, tamponnée phosphate à pH 7,4 à 25 °C ).
- On laisse le lyophilisat de polyaminoacide s'hydrater.
- Le gel obtenu se présente sous la forme d'une solution colloïdale visqueuse diffusant dans le bleu.

### 7.1.2 - ETAPE b) à e) : PREPARATION DES MICROCAPSULES :

- On procède tel que décrit dans l'exemple 4.1.2(i). Après centrifugation, on recueille 1 620 mg d'huile.

### 7.2 - CARACTERISATION DES MICROCAPSULES :

- La composition globale des microparticules avant redispersion est : 12% en polyaminoacide ; 38% en Miglyol ®; 50 % de solution saline.
- La répartition granulométrique des microparticules, mesurée par diffraction laser dans les conditions standard stipulées dans l'exemple 4, donne un diamètre moyen de référence **D[4,3] de 2,9 µm** avec un écart type de 1,5 µm (cf. **fig. 8,** courbe 1).

### LYOPHILISATION/REHYDRATATION :

- ***Test α***_{**1**} ***:***
   La dispersion de microparticules, ainsi obtenues et caractérisées, est lyophilisée pendant 48 heures. On récupère 212 mg de lyophilisat de microparticules. La réhydratation de la totalité de ce lyophilisât dans 5 ml de PBS, conduit, spontanément, à une suspension de microparticules présentant un profil granulomètrique quasiment superposable à celui d'avant l'étape de lyophilisation. Le D[4,3] est de 3,0 µm avec un écart type de 1,8 µm. (cf. **fig. 7,** courbe 2), soit un Δ D[4,3] par rapport au D[4,3] de référence à 3,4 %.

### EXEMPLE 8 : MICROPARTICULES NEUTRES PREPAREES A PARTIR D'HUILE DE SILICONE D'EAU ET POLY LEUCINE-CO-GLUTAMATE DE SODIUM DE COMPOSITION 30/70 ET DE STRUCTURE RANDOM.

### 8.1 - METHODOLOGIE :

### 8.1.1 - ETAPE A) : PREPARATION DU GEL :

- On introduit dans un tube à hémolyse 50 mg de lyophilisat de poly leucine-co-glutamate de sodium (noté LEU/GLU) de composition 30/70, synthétisé selon le protocole donné à l'exemple 1 en modifiant le ratio des monomères.
- On ajoute la phase aqueuse, typiquement 450 mg, de PBS 0,01 M (solution isotonique, tamponnée phosphate à pH 7,4 à 25 °C).
- On laisse le lyophilisat de polyaminoacide s'hydrater.
- Le gel obtenu se présente sous la forme d'une solution colloïdale visqueuse diffusant dans le bleu.

### 8.1.2 - ETAPE b) à e) : PREPARATION DES MICROCAPSULES :

- On introduit dans le tube à hémolyse contenant le gel de polyaminoacide 2 000 µl d'huile de silicone de référence Rhodorsil® 47V20.
- On agite le mélange avec un homogénéisateur du type rotor/stator (ULTRA-TURRAX T8, IKA LABORTECHNIK). On obtient une dispersion d'aspect laiteux de microparticules dans une phase continue huileuse.
- Le tube à hémolyse, contenant la dispersion de microparticules, est centrifugé (20 min à 3 500 tr/min). La phase surnageante, composée d'huile de silicone excédentaire, est séparée du sédimentat par simple écoulement ; on recueille ainsi 921 mg d'huile.
- Le sédimentat est redispersé dans de d'eau désionisée contenant du Thimérosal® (50 µg/ml), ou dans une solution saline tamponnée, par exemple du PBS 0,01 M (tampon phosphate, pH 7,4 à 25 °C) contenant du Thimérosal® (50 µg/ml). Le volume total de la dispersion obtenue est de 5 000 µl.

### 8.2 - CARACTERISATION DES MICROCAPSULES :

- La composition globale des microparticules avant redispersion est : 3,3 % en polyaminoacide ; 66,6 % de Rhodorsil® ; 30,1% de solution saline.
- La répartition granulométrique des microparticules, mesurée par diffraction laser dans les conditions standard stipulées dans l'exemple 4, donne un diamètre moyen D[4,3] qui est de 4,7 µm avec un écart type de 1,7 µm.

### EXEMPLE 9 : MICROPARTICULES NEUTRES PREPAREES A PARTIR D'HUILE DE DIFFERENTES NATURES D'EAU ET POLY LEUCINE-CO-GLUTAMATE DE SODIUM DE COMPOSITION 50/50 ET DE STRUCTURE RANDOM.

### 9.1 - METHODOLOGIE :

On procède comme indiqué dans l'exemple 8 et on met en oeuvre les huiles suivantes :

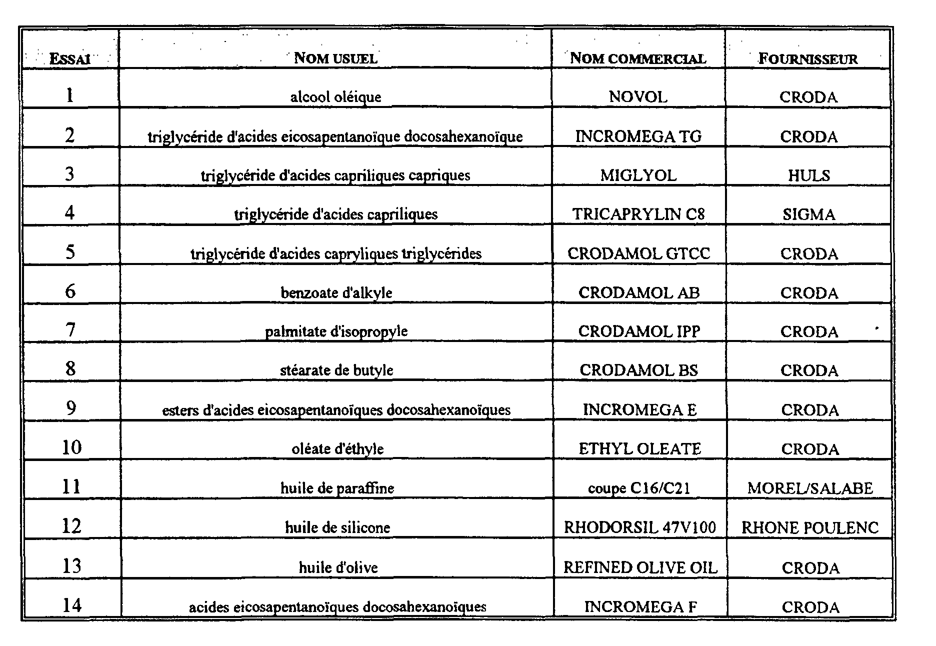

### 9.2 - CARACTERISATION DES MICROCAPSULES :

- La composition globale des microparticules, avant redispersion, varie selon les phases lipidiques utilisées, dans les intervalles suivants : 3 à 4 % en polyaminoacide ; 64 à 69 % huile, 28 à 32 % d'eau ou de solution saline. Seules les microparticules à base d'INCROMEGA E affichent une composition différente avant redispersion : 5 % en polyaminoacide ; 51 % huile ; 44 % d'eau ou de solution saline.
- Les répartitions granulométriques des microparticules sont mesurées par diffraction laser. L'appareil utilisé est un COULTER LS130 ; le modèle de calcul choisi est le modèle de FRAUNHOFER inclued "PIDS". Les diamètres moyens volumiques, D[4,3], et les écarts types obtenus sont reportés sur le graphe de la **fig. 8.**

### EXEMPLE 10 : MICROPARTICULES NEUTRES PREPAREES A PARTIR DE MIGLYOL®, D'EAU ET DE DIFFERENTS COPOLY α-AMINOACIDES DE STRUCTURE RANDOM.

### 10.1 - METHODOLOGIE :

On procède comme mentionné dans l'exemple 4.1.1 et 4.1.2(i). Les copoly α-aminoacides mis en oeuvre sont ceux donnés dans le tableau ci-dessous. Le temps d'hydratation du lyophilisât de polymère dans la solution isotonique (PBS 0,01M tamponnée à pH 7,4) est de 24 heures. Les gels obtenus présentent des viscosités différentes.

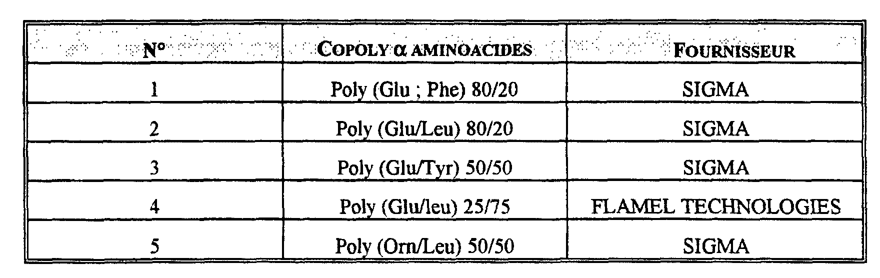

### 10.2 - CARACTERISATION DES MICROPARTICULES :

- La composition globale des microparticules, avant redispersion, varie selon les polymères utilisés, dans les intervalles suivants 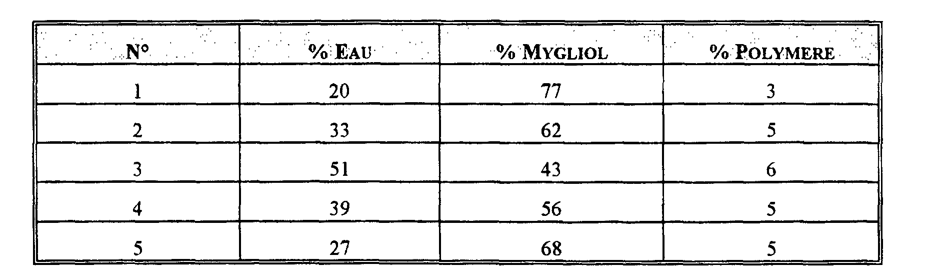
- Les répartitions granulométriques des microparticules sont mesurées par diffraction laser. L'appareil utilisé est un COULTER LS130 ; le modèle de calcul choisi est le modèle de FRAUNHOFER inclued "PIDS". Les diamètres moyens volumiques, D[4,3], et les écarts types (SD) obtenus sont reportés sur le tableau suivant : 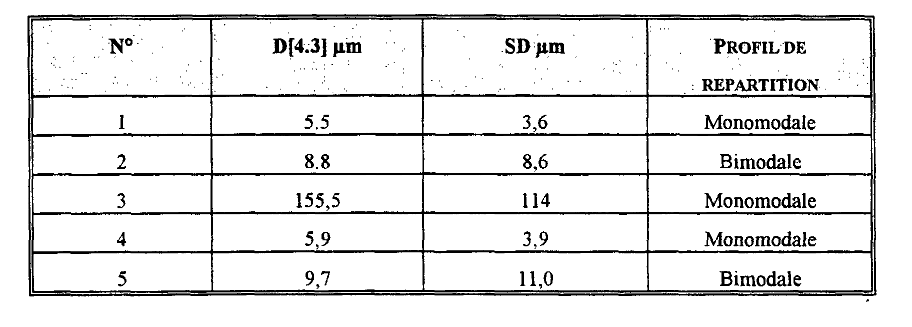
- Les microparticules des lots 1 à 5 satisfont au test α₂.

### EXEMPLE 11 : MICROPARTICULES CONTENANT DU CYTOCHROME, PREPAREES A PARTIR DE MIGLYOL®, D'EAU ET DE POLY LEUCINE-CO-GLUTAMATE DE SODIUM DE COMPOSITION 50/50 ET DE STRUCTURE RANDOM - ENCAPSULATION PAR VOIE DIRECTE.

### 11.1 - METHODOLOGIE :

Trois lots de microcapsules ont été fabriquées selon des conditions opératoires identiques décrites ci-dessous.

### 11.1.1 - ETAPE α) : PREPARATION DU GEL :

- On prépare 1,5 ml d'une solution mère de cytochrome C coeur de cheval (ref. C2506 SIGMA) à 60 mg/ml dans un tampon phosphate isotonique (PBS) 0,01 M, pH 7,4.
- On introduit, dans un tube à hémolyse, 50 ± 0,2 mg de lyophilisat de poly leucine-co-glutamate de sodium (noté LEU/GLU) de composition 50/50, de structure random et de M_{w} de 110 000.
- On ajoute 450 mg de la solution mère de cytochrome,
- On laisse le lyophilisât de polyaminoacide s'hydrater.
- Le gel obtenu se présente sous la forme d'une solution colloïdale visqueuse rouge/orangé.

### 11.1.2 - ETAPES b) à e) : PREPARATION DES MICROCAPSULES SELON PROTOCOLE EXEMPLE 4.1.2(I) :

- On introduit, dans le tube à hémolyse contenant le gel de polyaminoacide et de cytochrome, 2 000 µl de Miglyol® (DYNAMIT NOBEL).
- On agite le mélange avec un homogénéisateur du type rotor/stator (ULTRA-TURRAX T8, IKA LABORTECHNIK). On obtient une dispersion d'aspect laiteux de microparticules dans une phase continue lipidique.
- Le tube à hémolyse, contenant la dispersion de microparticules, est centrifugé. La phase surnageante, composée de Miglyol® excédentaire, est séparée du sédimentat par simple écoulement. On recueille ainsi 1 577 mg d'huile.
- Le sédimentat est redispersé dans du PBS filtrée à 0,22 µm contenant du Thimérosal® (50 µg/ml). Le volume total de la dispersion obtenue est de 20 ml. •Stockage de la dispersion de microparticules dans une enceinte ventilée, conditionnée à la température de 37 °C.

### 11.2 - CARACTERISATION DES MICROPARTICULES :

- La composition globale des trois lots de microparticules avant redispersion varie de : 5 ± 1 % en polyaminoacide ; 39 ± 3 % en Miglyol® ; 55 ± 3 % en PBS.
- La répartition granulométrique des microparticules, mesurée par diffraction laser sur un COULTER LS130 en mode FRAUNHOFER inclued "PIDS", est monomodale pour les trois lots, avec un diamètre moyen volumique **D[4,3] de 2,4 ± 0,5 µm** et un écart type de 1,1 ± 0,4 µm.

### 11.3 - ETUDE DE RELARGAGE :

Pour l'étude de relargage on procède par filtration, à un temps donné, d'un volume connu de dispersion de microparticules. Le filtrat est ensuite analysé par HPLC, pour doser le cytochrome non encapsulé ou relargué. Les filtrations ont été réalisées à T₀ puis après 3 heures, 5 heures, 17 heures, 24 heures et 5 jours de stockage à 37 °C.
- *CONDITIONS DE FILTRATION :*
   - Cellule de diafiltration à agitation de type AMICON de 50 ml de capacité.
   - Membrane de type polysulfone (réf. XM 300, AMICON).
- *CONDITIONS DE DOSAGE :*
   - HPLC à séparation par affinité sur colonne PLRPS (300 A).
- *RESULTATS :*
   - Les rendements d'encapsulation, définis comme le rapport de la quantité de cytochrome encapsulé sur la quantité de cytochrome initialement introduite, sont de 100 %, pour un taux d'encapsulation de 35 % de cytochrome/polymère.
   - On n'observe aucun relargage R de cytochrome dans le milieu de dispersion, après 5 jours de stockage à 37 °C (cf. **fig. 9).**

### EXEMPLE 12 : MICROPARTICULES CONTENANT DU CYTOCHROME, PREPAREES A PARTIR DE MIGLYOL®, D'EAU ET DE POLY LEUCINE-CO-GLUTAMATE DE SODIUM DE COMPOSITION 50/50 ET DE STRUCTURE RANDOM - ENCAPSULATION PAR AUTO ASSOCIATION.

### 12.1 - METHODOLOGIE :

Un lot de microcapsules a été fabriqué selon les conditions opératoires décrites dans l'exemple 4.1.1 et 4.1.2(i). Le sédimentat de microparticule est alors dispersé dans un volume de 10 ml de solution isotonique tamponné phosphate à pH 7,4 contenant 1,0 mg/ml de cytochrome C coeur de cheval. Après un temps de contact de 24 h à température ambiante, la dispersion de microcapsules est filtrée selon les conditions données dans l'exemple 11. L'analyse HPLC révèle une concentration en cytochrome libre présent dans le filtrat de 0,5 mg/ml.

### 12.2 - RESULTAT :

Le taux d'encapsulation en cytochrome est de 10 % par rapport au polymère, pour un rendement d'encapsulation de 50%.

### EXEMPLE 13 : MICROPARTICULES CONTENANT UNE PROTEINE ANTIGENIQUE DE LA MALADIE DE LYME "OSP A", PREPAREES A PARTIR DE MIGLYOL®, D'EAU ET DE POLY EUCINE-CO-GLUTAMATE DE SODIUM DE COMPOSITION 50/50 ET DE STRUCTURE RANDOM.: ENCAPSULATION PAR VOIE DIRECTE.

### 13.1 - METHODOLOGIE :

### 13.1.1 - ETAPE a): PREPARATION DU GEL :

- On introduit dans un tube à hémolyse 50 mg de lyophilisât de poly leucine-co-glutamate de sodium (noté LEU/GLU) synthétisé selon l'exemple 1.
- On ajoute 450 mg d'une solution aqueuse contenant 4,4 mg/ml d'Osp A.

### 13.1.2 - ETAPES b) à e) : PREPARATION DES MICROCAPSULES :

- On effectue ces étapes de la même facçon que celle décrite pour le procédé de l'exemple 4.1.2(i).
*FILTRATION :*
- Un volume de 2 ml de dispersion de microcapsules est introduit dans un tube d'ultrafiltration de type ULTRAFREE XM300, commercialisé par MILLIPORE.
- Après centrifugation, on recueille 1 ml de filtrat que l'on analyse par dosage ELISA.

### 13.2 - RESULTAT :

- Le dosage ELISA révèle une concentration d'Osp A, non encapsulée, dans le filtrat de 0,21 mg/ml.
- Le taux d'encapsulation en Osp A est de 0,36% par rapport au polymère, pour un rendement d'encapsulation de 46 %.

### EXEMPLE 14 : MICROPARTICULES CONTENANT UNE GLYCOPROTEINE ANTIGENIQUE DE LA MALADIE DE L'HERPES "gD2t", PREPAREES A PARTIR DE MIGLYOL®, D'EAU ET DE POLYLEUCINE-CO-GLUTAMATE DE SODIUM DE COMPOSITION 50/50 ET DE STRUCTURE RANDOM - ENCAPSULATION PAR VOIE DIRECTE.

### 14.1 - METHODOLOGIE :

### 14.1.1 - ETAPE α) : PREPARATION DU GEL :

- On introduit, dans un tube à hémolyse, 50 mg de lyophilisat de poly leucine-co-glutamate de sodium (noté LEU/GLU) selon l'exemple 1.
- On ajoute 450 ± 0,2 mg d'une solution aqueuse contenant 0,666 mg/ml de gD2t.

### 14.1.2 - ETAPES b) à e) : PREPARATION DES MICROCAPSULES:

- On effectue ces étapes de la même façon que décrite pour le procédé avec inversion d'émulsion de l'exemple 4.1.2(i).
*FILTRATION :*
- Un volume de 2 ml de dispersion de microcapsules est introduit dans un tube d'ultrafiltration de type ULTRAFREE XM300, commercialisé par MILLIPORE.
- Après centrifugation, on recueille 1 ml de filtrat que l'on analyse par dosage ELISA.

### 14.2 - RESULTATS :

- Le dosage ELISA révèle une concentration de gG2t, non encapsulée, dans le filtrat de 5 µg/ml.
- Le taux d'encapsulation "gD2t" est de 0,12% par rapport au polymère, pour un rendement d'encapsulation de 92 %.

## Revendications

1. Microparticules susceptibles d'être utilisées comme vecteurs de principe(s) actif(s) **(PA),** caractérisées :
* α * en ce qu'elles ont chacune une structure cohésive faite de gel-composite, physico-chimiquement stable et intègre,
* β * en ce qu'elles tirent leur cohésion de la présence des trois composés suivants :
**(I)** de l'huile,
**(II)** une phase aqueuse,
**(III)** et au moins un copolyaminoacide linéaire non réticulé, synthétique et comportant au moins deux types différents de comonomères aminoacides AAᵢ hydrophile et AAₒ hydrophobe,
* γ * et en ce qu'elles présentent une taille moyenne contrôlable et ajustable sur une gamme inférieure ou égale à 500 µm, de préférence à 200 µm et, plus préférentiellement, comprise entre 0,05 et 100 µm.

2. Microparticules selon la revendication 1, **caractérisées en ce que** leurs caractéristiques * α * sont reflétées par au moins l'une des propriétés fonctionnelles suivantes :
- α₁ - absence de coalescence des microparticules suite à un traitement de lyophilisation, cette non-coalescence se traduisant par une conservation de la répartition granulométrique, en particulier du D[4,3] à raison de ± 20 %, après réhydratation selon un test α₁ ;
- α₂ - absence de coalescence à la centrifugation se traduisant par une conservation de la répartition granulométrique et, en particulier du D[4,3] à raison de ± 20 %, selon un test α₂ ;
- α₃ - résistance aux variations de pH se traduisant par une conservation de la répartition granulométrique des microparticules et, en particulier, du D[4,3] à raison de ± 20 %, après soumission à des pH de 2 à 13, selon un test α₃ ;
- α₄ - absence de coalescence en dispersion dans une solution saline tamponnée, se traduisant par une conservation de la répartition granulométrique des microparticules, en particulier de leur D[4,3] à raison de ± 20 %, pendant des durées de stockage supérieures ou égales à 3, 9 et 18 mois à des températures de 37°C, ambiante et 4°C, respectivement, selon un test α₄.

3. Microparticules selon la revendication 1 ou 2, **caractérisées en ce que** le copolyaminoacide (III) présente :
* des comonomères AAᵢ, choisis dans le groupe d'aminoacides suivants : acide glutamique, acide aspartique, omithine, arginine, lysine, asparagine, histidine et leurs mélanges,
* ainsi que des comonomères AAₒ choisis dans le groupe d'aminoacides suivants : leucine, tyrosine, phénylalanine, valine, cystine, isoleucine et leurs mélanges.

4. Microparticules selon la revendication 3, **caractérisées en ce que** AAᵢ = Glu et/ou Asp et AAₒ = Leu et/ou Isoleu, et/ou Tyr et/ou Phe.

5. Microparticules selon l'une quelconque des revendications 1 à 4, **caractérisées en ce que** le copolyaminoacide **(III)** est de structure statistique ou de structure dibloc, tribloc ou multibloc.

6. Microparticules selon l'une quelconque des revendications 1 à 5, **caractérisées en ce que** le copolyaminoacide **(III)** est soit multibloc et possède une masse molaire M_{w}, supérieure ou égale à 5 000 D, de préférence comprise entre 5 000 et 100 000 D et, plus préférentiellement encore, entre 5 000 et 20 000 D, soit random (statistique) et possède une masse molaire M_{w}, supérieure ou égale à 50 000 D, de préférence comprise entre 10 000 et 300 000 D et, plus préférentiellement encore, comprise entre 10 000 à 100 000 D.

7. Microparticules selon l'une quelconque des revendications 1 à 6, **caractérisées en ce que** l'huile (I) est formée par un ou plusieurs composés gras sélectionnés dans le groupe suivant :
* triglycéride(s) d'acide d'ester gras à chaînes moyennes d'origine animale, végétale ou synthétique,
* paraffine(s),
* huile(s) polysiloxane(s),
* acides gras d'origine animale ou végétale,
* esters acides gras, leurs esters et/ou leurs sels.

8. Microparticules selon l'une quelconque des revendications 1 à 7, **caractérisées en ce que** la phase aqueuse **(II)** est constituée par une solution saline, de préférence tamponnée.

9. Microparticules selon l'une quelconque des revendications 1 à 8, **caractérisées par** la composition suivante :
**(I)** triglycéride(s) d'acide d'ester gras à chaînes moyennes, l'huile de coco étant particulièrement préférée,
**(II)** eau déionisée ou solution saline tamponnée, le pH de cette phase aqueuse étant compris entre 6 et 8,
**(III)** copolyaminoacide de type Leu/Glu, de préférence 50/50.

10. Procédé de préparation de microparticules, notamment du type de celles selon l'une quelconque des revendications 1 à 9, **caractérisé en ce qu'**il comprend, essentiellement, les étapes suivantes, successives ou non :
- **a** -préparation d'un gel à partir de la phase aqueuse **(II)** et d'au moins un copolyaminoacide **(III), (II)** et **(III)** étant tels que définis dans les revendications 1 à 9,
- **b** -mise en présence du gel issu de l'étape **- a -** avec de l'huile **(I),** telle que définie dans les revendications 1 et/ou 7,
- **c -** agitation du mélange gel + **(I)** conduisant à une dispersion de microparticules dans une phase continue huileuse **(I)** ou aqueuse **(II),**
- **d** -éventuelle séparation des microparticules et de la phase continue huileuse ou aqueuse, de préférence par centrifugation,
- **e -** éventuelle redispersion des microparticules recueillies à l'issue de l'étape **- e -** dans un liquide de stockage,
- **f -** éventuel traitement de lyophilisation des microparticules de l'étape **- d -**, redispersées ou non.

11. Procédé selon la revendication 10, **caractérisé en ce que** l'étape **- a -** consiste à mélanger la phase aqueuse **(II)** et le (ou les) copolyaminoacide(s) **(III)** dans des proportions telles que **(III)** représente de 2 à 50 %, de préférence de 5 à 30 % en poids du gel **(III)** + **(II).**

12. Procédé selon la revendication 10 ou la revendication 11, **caractérisé en ce que** l'on prévoit d'introduire de l'huile **(I)** dans le mélange de l'étape **- b -,** au cours de l'étape **- c -**.

13. Procédé selon l'une quelconque des revendications 10 à 12, **caractérisé en ce que** l'agitation **- c -** est réalisée à l'aide d'un dispositif rotor/stator et en mettant en oeuvre une vitesse d'agitation comprise entre 1 000 et 40 000, de préférence 5 000 et 25 000 tr/min.

14. Procédé selon l'une quelconque des revendications 10 à 13, **caractérisé en ce qu'**il s'effectue à température ambiante comprise, de préférence, entre 10 et 35°C.

15. Procédé selon l'une quelconque des revendications 10 à 14, **caractérisé en ce que** l'on incorpore au moins un **PA** dans l'huile **(I)** et/ou dans la phase aqueuse **(II)** et/ou dans le **PAA (III)** et/ou dans le gel **(II)** + **(III)** de l'étape **- a -**, de facon à obtenir des microparticules chargées en **PA**.

16. Microparticules selon l'une quelconque des revendications 1 à 9 et/ou obtenues par le procédé selon l'une quelconque des revendications 10 à 15,
**caractérisées en ce que** le principe actif est médicamenteux et, de préférence, choisi parmi :
* les protéines et/ou les peptides parmi lesquels les plus préférentiellement retenus sont : les hémoglobines, les cytochromes, les albumines, les interférons, les antigènes, les anticorps, la calatonine, l'érythropoïétine, l'insuline, les hormones de croissance, le facteur IX, l'interleukine ou leurs mélanges,
* les polysaccharides, l'héparine étant plus particulièrement sélectionnée,
* les acides nucléiques et, préférablement, les oligonucléotides d'ARN et/ou d'ADN,
* et leurs mélanges.

17. Microparticules selon l'une quelconque des revendications 1 à 9 et/ou obtenues par le procédé selon l'une quelconque des revendications 10 à 15, **caractérisées en ce que** le principe actif est constitué par au moins un vaccin.

18. Spécialité pharmaceutique pour administration, de préférence, par voie orale, nasale, vaginale, oculaire, sous-cutanée, intraveineuse, intramusculaire, intradermique, intrapéritonéale, intracérébrale ou parentérale,
**caractérisée en ce qu'**elle comporte des particules selon l'une quelconque des revendications 1 à 9, 16 et 17 et/ou obtenues par le procédé selon l'une quelconque des revendications 10 à 15.

19. Microparticules selon l'une quelconque des revendications 1 à 9 et/ou obtenues par le procédé selon l'une quelconque des revendications 10 à 15, **caractérisées en ce que** le principe actif est un produit phytosanitaire ou cosmétique.

## Patentansprüche

1. Mikroteilchen, die als Vektoren von Wirkstoff/en (PA) verwendbar sind, **dadurch gekennzeichnet**,
* α* dass sie jeweils eine kohäsive Struktur besitzen, die von physikalisch-chemisch stabilem und integrem Verbundgel gebildet ist,
* β* dass sie ihre Kohäsion aus dem Vorliegen der drei folgenden Verbindungen ziehen:
(I) Öl,
(II) eine wässrige Phase,
(III) und mindestens eine unverzweigte, nicht vernetzte synthetische Copolyaminosäure mit mindestens zwei verschiedenen Typen von Aminosäurecomonomeren AAᵢ hydrophil und AA₀ hydrophob,
* γ* dass sie eine steuerbare mittlere Größe besitzen, die auf einem Bereich kleiner oder gleich 500 *µ*m, vorzugsweise kleiner oder gleich 200 *µ*m, und noch bevorzugter zwischen 0,05 und 100 *µ*m, einstellbar ist.

2. Mikroteilchen nach Anspruch 1, **dadurch gekennzeichnet, dass** ihre Merkmale *α* von mindestens einer der folgenden funktionellen Eigenschaften wiedergespiegelt werden:
- α₁- Fehlen von Koaleszenz der Mikroteilchen auf eine Lyophilisationsbehandlung hin, wobei dieses Nichtkoaleszenz sich in einer Beibehaltung der Korngrößenverteilung, insbesondere des D[4,3] von ± 20%, nach Rehydratisierung gemäß einem Test α₁ äußert;
- α₂- Fehlen von Koaleszenz bei Zentrifugation, was sich in einer Beibehaltung der Korngrößenverteilung und insbesondere des D[4,3] von ± 20% gemäß einem Test α₂ äußert;
- α₃- Festigkeit gegenüber pH-Schwankungen, die sich in einer Beibehaltung der Korngrößenverteilung der Mikroteilchen und insbesondere des D[4,3] von +20% äußert, nachdem sie pH-Werten von 2 bis 13 gemäß einem Test α₃ ausgesetzt wurden;
- α₄- Fehlen von Koaleszenz in Dispersion in einer gepufferten Salzlösung, was sich in einer Beibehaltung der Korngrößenverteilung der Mikroteilchen, insbesondere ihres D[4,3] von ± 20% während Lagerzeiten von gleich 3, 9 und 18 Monaten oder mehr bei Temperaturen von 37°C, Raumtemperatur bzw. 4°C gemäß einem Test α₄ äußert.

3. Mikroteilchen nach Anspruch 1 oder 2, **dadurch gekennzeichnet, dass** die Copolyaminosäure (III) folgendes aufweist:
* Comonomere AAᵢ, die aus der Gruppe der folgenden Aminosäuren ausgewählt sind: Glutaminsäure, Asparginsäure, Ornithin, Arginin, Lysin, Asparagin, Histidin und ihre Mischungen,
* sowie Comonomere AA₀, die aus der Gruppe der folgenden Aminosäuren ausgewählt sind: Leucin, Tyrosin, Phenylalanin, Valin, Cystin, Isoleucin und ihre Mischungen.

4. Mikroteilchen nach Anspruch 3, **dadurch gekennzeichnet, dass** AAᵢ = Glu und/oder Asp und AA₀ = Leu und/oder Isoleu und/oder Tyr und/oder Phe sind.

5. Mikroteilchen nach einem der Ansprüche 1 bis 4, **dadurch gekennzeichnet, dass** die Copolyaminosäure (III) eine statistische Struktur oder eine Zweiblock-, Dreiblock- oder Mehrblockstruktur aufweist.

6. Mikroteilchen nach einem der Ansprüche 1 bis 5, **dadurch gekennzeichnet, dass** die Copolyaminosäure (III) entweder Mehrblockstruktur aufweist und eine Molmasse M_{w} größer oder gleich 5 000 D, vorzugsweise zwischen 5 000 und 100 000 D und noch bevorzugter zwischen 5 000 und 20 000 D besitzt oder random (statistisch) ist und eine Molmasse M_{w} größer oder gleich 50 000 D, vorzugsweise zwischen 10 000 und 300 000 D und noch bevorzugter zwischen 10 000 bis 100 000 D besitzt.

7. Mikroteilchen nach einem der Ansprüche 1 bis 6, **dadurch gekennzeichnet, dass** das Öl (I) von einer oder mehreren Fettverbindungen gebildet ist, die aus der folgenden Gruppe ausgewählt sind:
* Fettsäureestertriglycerid(e) mit mittleren Ketten tierischen, pflanzlichen oder synthetischen Ursprungs,
* Parafin(e),
* Polysiloxanöl(e),
* Fettsäuren tierischen oder pflanzlichen Ursprungs,
* Fettsäureester, ihre Ester und/oder ihre Salze.

8. Mikroteilchen nach einem der Ansprüche 1 bis 7, **dadurch gekennzeichnet, dass** die wässrige Phase (II) aus einer vorzugsweise gepufferten Salzlösung besteht.

9. Mikroteilchen nach einem der Ansprüche 1 bis 8, **gekennzeichnet durch** die folgende Zusammensetzung:
(I) Fettsäureestertriglycerid(e) mit mittleren Ketten, wobei Kokosöl besonders bevorzugt wird,
(II) entionisiertes Wasser oder gepufferte Salzlösung, wobei das pH dieser wässrigen Phase 6 bis 8 beträgt,
(III) Copolyaminosäure vom Typ Leu/Glu, vorzugsweise 50/50.

10. Verfahren zur Herstellung von Mikroteilchen, insbesondere von Mikroteilchen nach einem der Ansprüche 1 bis 9, **dadurch gekennzeichnet, dass** es im wesentlichen die folgenden Schritte, aufeinanderfolgend oder nicht, umfasst:
- a- Herstellung eines Gels aus der wässrigen Phase (II) und mindestens einer Copolyaminosäure (III), wobei (II) und (III) der Definition in den Ansprüche 1 bis 9 entsprechen,
- b- In-Kontakt-Bringen des aus Schritt -a- hervorgegangenen Gels mit Öl (I) gemäß den Ansprüche 1 und/oder 7,
- c- Rühren der Mischung Gel + (I), was zu einer Dispersion von Mikroteilchen in einer kontinuierlichen Ölphase (I) oder wässrigen Phase (II) führt,
- d- ggf. Trennen der Mikroteilchen und der kontinuierlichen Ölphase oder wässrigen Phase vorzugsweise durch Zentrifugieren,
- e- ggf. Redispersion der am Ende von Schritt -d- gewonnenen Mikroteilchen in einer Lagerflüssigkeit,
- f- ggf. Lyophilisationsbehandlung der redispergierten oder nicht redispergierten Mikroteilchen von Schritt -d-.

11. Verfahren nach Anspruch 10, **dadurch gekennzeichnet, dass** der Schritt -a- darin besteht, dass die wässrige Phase (II) und die Copolyaminosäure(n) (III) in solchen Anteilen gemischt werden, dass (III) 2 bis 50 Gew.-%, vorzugsweise 5 bis 30 Gew.-%, des Gels (III)+(II) darstellt.

12. Verfahren nach Anspruch 10 oder Anspruch 11, **dadurch gekennzeichnet, dass** man vorsieht, Öl (I) in die Mischung von Schritt -b- während des Schritts -c- einzuführen.

13. Verfahren nach einem der Ansprüche 10 bis 12, **dadurch gekennzeichnet, dass** das Rühren -c- mit Hilfe einer Rotor/Stator-Vorrichtung und unter Einsetzen einer Rührgeschwindigkeit zwischen 1 000 und 40 000, vorzugsweise zwischen 5 000 und 25 000 U/min vorgenommen wird.

14. Verfahren nach einem der Ansprüche 10 bis 13, **dadurch gekennzeichnet, dass** es bei Raumtemperatur vorzugsweise zwischen 10 und 35°C stattfindet.

15. Verfahren nach einem der Ansprüche 10 bis 14, **dadurch gekennzeichnet, dass** man einen PA in das Öl (I) und/oder in die wässrige Phase (II) und/oder in die PAA (III) und/oder in das Gel (II)+(III) von Schritt -a- so einführt, dass mit PA chargierte Mikroteilchen erhalten werden.

16. Mikroteilchen nach einem der Ansprüche 1 bis 9 und/oder mit dem Verfahren nach einem der Ansprüche 10 bis 15 erhaltene Mikroteilchen,
**dadurch gekennzeichnet, dass** der Wirkstoff ein Arzneimittel ist und vorzugsweise aus den folgenden Substanzen ausgewählt ist:
* Proteine und/oder Peptide, von denen am meisten bevorzugt werden: Hämoglobine, Cytochrome, Albumine, Interferone, Antigene, Antikörper, Calatonin, Erythropoetin, Insulin, Wachstumshormone, Faktor IX, Interleukin oder ihre Mischungen,
* Polysaccharide, wobei insbesondere Heparin gewählt wird,
* Nukleinsäuren und vorzugsweise die Oligonukleotide RNA und/oder DNA,
* und ihre Mischungen.

17. Mikroteilchen nach einem der Ansprüche 1 bis 9 und/oder mit dem Verfahren nach einem der Ansprüche 10 bis 15 erhaltene Mikroteilchen, **dadurch gekennzeichnet, dass** der Wirkstoff aus mindestens einem Impfstoff besteht.

18. Pharmazeutikum zur Verabreichung vorzugsweise auf oralem, nasalem, vaginalem, okularem, subkutanem, intravenösem, intramuskulärem, intradermalem, intraperitonealem, intracerebralem oder parenteralem Weg,
**dadurch gekennzeichnet, dass** es Teilchen nach einem der Ansprüche 1 bis 19, 16 und 17 oder mit dem Verfahren nach einem der Ansprüche 10 bis 15 erhaltene Teilchen umfasst.

19. Mikroteilchen nach einem der Ansprüche 1 bis 9 und/oder mit dem Verfahren nach einem der Ansprüche 10 bis 15 erhaltene Teilchen, **dadurch gekennzeichnet, dass** der Wirkstoff ein Pflanzenpflegeprodukt oder ein kosmetisches Produkt ist.

## Claims

1. Microparticles for use as active principle(s) (PA) vectors **characterized in that**:
* α* each one comprises a cohesive structure made of physicochemically stable and integral composite gel,
* β* their cohesion is due to the presence of the three following compounds:
(I) oil,
(II) aqueous phase,
(III) and at least one linear noncrosslinked, synthetic copolyaminoacid, comprising at least two different types of aminoacid comonomers, a hydrophilic comonomer AAᵢ and a hydrophobic comonomer AAₒ, and
* γ* they have an average size, which is controllable and adjustable over a range less than or equal to 500 µm, preferentially 200 µm, and more preferentially, comprised between 0,05 and 100 µm.

2. Microparticles according to claim 1, wherein *α* characteristics are reflected by at least one of the following properties:
- α₁- absence of coalescence of the microparticles after a lyophilization treatment, this non-coalescence being reflected by a conservation of the particle size distribution, in particular of the D[4,3] in a proportion of +/- 20 %, after rehydratation according to a α₁ test;
- α₂- absence of coalescence on centrifugation, reflected by a conservation of the particle size distribution, in particular of the D[4,3] in a proportion of +/- 20 %, after rehydratation according to a α₂ test;
- α₃- resistance to pH variations, reflected by a conservation of the particle size distribution, in particular of the D[4,3] in a proportion of +/- 20 %, after exposure to pH of 2 to 13, according to a α₃ test;
- α₄- absence of coalescence in dispersion in a buffered saline solution, reflected by a conservation of the particle size distribution, in particular of the D[4,3] in a proportion of +/- 20 %, for storage times of greater than or equal to 3, 9, and 18 months at temperatures of 37 °C, ambient and 4 °C, respectively, according to a α₄ test.

3. Microparticles according to claim 1 or 2, wherein the copolyaminoacid (III) comprises:
* comonomers AAᵢ, selected from the following aminoacids group: glutamic acid, aspartic acid, ornithine, arginine, lysine, asparagine, histidine and mixtures thereof,
* comonomers AAₒ, selected from the following aminoacids group: leucine, tyrosine, phenylalanine, valine, cystine, isoleucine, and mixtures thereof.

4. Microparticles according to claim 3, wherein AAᵢ = Glu and/or Asp and AAₒ = Leu and/or Isoleu, and/or Tyr, and/or Phe.

5. Microparticles according to any of claim I to 4, wherein the copolyaminoacid (III) is of random structure, or of diblock, triblock or multiblock structure.

6. Microparticles according to any of claim 1 to 5, wherein copolyaminoacid (III) is either multibloc and have a molar mass M_{w}, greater than or equal to 5 000 D, preferentially from 5 000 to 100 000 D and, more preferentially from 5 000 to 20 000 D, or random and have a molar mass M_{w}, greater than or equal to 50 000 D, preferentially from 10 000 to 300 000 D and, more preferentially from 10 000 to 100 000 D.

7. Microparticles according to any of claim 1 to 6, wherein oil **(I)** is formed by one or several fatty compounds of the following group:
* medium-chain fatty ester acid triglyceride(s) of animal, plant or synthetic origin,
* paraffin(s),
* polysiloxane oil(s),
* fatty acids of animal or plant origin, and
* fatty acids esters, esters and/or salts thereof.

8. Microparticles according to any of claim 1 to 7, wherein aqueous phase (II) comprises a saline solution, preferably a buffered one.

9. Microparticles according to any of claim 1 to 8, comprising:
(I) medium-chain fatty ester acid triglyceride(s), coco oil being particularly preferred,
(II) deionised water or buffered saline solution, pH of this aqueous phase being between 6 and 8,
(III) copolyaminoacid of Leu/Glu type, preferably 50/50.

10. Process for the preparation of microparticles according to any of claims 1 to 9, comprising the successive or not following steps of:
- a - - preparing a gel from the aqueous phase (II) and at least one said copolyaminoacid (III), (II) and (III) being defined in claims 1 to 9;
- b -- placing the gel obtained from step -a- in contact with oil (I), as defined in claims 1 and/or 7;
- c -- stirring the gel + (I) mixture to form a dispersion of microparticles in an oily (I) or aqueous (II) continuous phase;
- d - optionally separating the microparticles and the oily or aqueous continuous phase, preferably by centrifugation ;
- e -- optionally redispersing the separated microparticles from step -e- in a storage liquid; and
- f - optionally lyophilizing the microparticles from step d), redispersed or not.

11. Process according to claim 10, wherein step a) comprises mixing the aqueous phase (II) and the at least one copolyaminoacid (III) in proportions such that the at least one copolyaminoacid (III) represents from 2 to 50%, preferable from 5 to 30 % by weight of the gel (III) + (II).

12. Process according to claim 10 or 11, wherein during step c), the oil (I) is introduced into the mixture from step -b-.

13. Process according to any of claims 10 to 12, wherein the stirring -c- is carried out using a rotor/stator device and working at a stirring speed of between 1 000 and 40 000 rpm, preferably between 5 000 and 25 000 rpm.

14. Process according to any of claims 10 to 13, carried out at room temperature, preferably between 10 and 35°C.

15. Process according to any of claims 10 to 14, wherein at least one active principle PA is incorporated into the oil (I) and/or into the aqueous phase (II) and/or PAA (III) and/or into the gel (II) + (III) from step -a-, so as to obtain microparticles filled with PA.

16. Microparticles according to any of claims 1 to 9, and /or obtained by process according to any of claims 10 to 15, comprising at least one active principle which is medicinal and preferably selected from the group consisting of:
* proteins and/or peptides and preferably: haemoglobins, cytochromes, albumins, interferons, antigens, antibodies, calatonin, erythropoïetin, insuline, growth hormones, factor IX, interleukin or mixtures thereof,
* polysaccharides, heparin being more particularly selected,
* nucleic acids and, preferably, RNA and/or DNA oligonucleotides,
* and mixtures thereof.

17. Microparticles according to any of claims 1 to 9, and /or obtained by process according to any of claims 10 to 15, comprising an active principle, which is at least one vaccine.

18. Pharmaceutical specialty for oral, nasal, vaginal, ocular, subcutaneous, intravenous, intramuscular, intradermal, intraperitoneal, intracerebral or parenteral administration, comprising particles according to any of claims 1 to 9, 16 and 17 and /or obtained by process according to any of claims 10 to 15.

19. Microparticles according to any of claims 1 to 9, and /or obtained by process according to any of claims 10 to 15, comprising at least one active principle, which is a phytosanitary or cosmetic product.
